(19) Europäisches Patentamt
European Patent Office
Office européen des brevets

(11) EP 2 512 236 B1

(12) **EUROPEAN PATENT SPECIFICATION**

(45) Date of publication and mention of the grant of the patent:
**19.10.2016 Bulletin 2016/42**

(21) Application number: **10838355.5**

(22) Date of filing: **20.12.2010**

(51) Int Cl.:
***A01N 37/00*** *(2006.01)* ***A61K 31/19*** *(2006.01)*

(86) International application number:
**PCT/US2010/061367**

(87) International publication number:
**WO 2011/075741 (23.06.2011 Gazette 2011/25)**

(54) **IMPROVED METHOD OF ADMINISTERING BETA-HYDROXY-BETA-METHYLBUTYRATE (HMB)**

VERBESSERTES VERFAHREN ZUR VERABREICHUNG VON BETA-HYDROXY-BETA-METHYLBUTYRAT (HMB)

PROCÉDÉ AMÉLIORÉ PERMETTANT D'ADMINISTRER DU BÊTA-HYDROXY-BÊTA-MÉTHYLBUTYRATE (HMB)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2009 US 287857 P**

(43) Date of publication of application:
**24.10.2012 Bulletin 2012/43**

(73) Proprietor: **Metabolic Technologies, Inc.**
**Ames, IA 50010 (US)**

(72) Inventors:
- **RATHMACHER, John**
  **Story City**
  **Iowa 50248 (US)**
- **FULLER, John**
  **Zearing**
  **Iowa 50278 (US)**
- **BAIER, Shawn**
  **Polk City**
  **Iowa 50226 (US)**
- **NISSEN, Steve**
  **Ames**
  **Iowa 50014 (US)**
- **ABUMRAD, Naji**
  **Nashville**
  **Tennessee 37220 (US)**

(74) Representative: **Evans, Jacqueline Gail Victoria**
**Marches Intellectual Property Limited**
**Wyastone Business Park**
**Wyastone Leys**
**Ganarew**
**Monmouth NP25 3SR (GB)**

(56) References cited:
**US-A- 4 992 470** **US-A- 5 028 440**
**US-A- 5 087 472** **US-A- 6 031 000**
**US-A1- 2004 220 266** **US-A1- 2005 215 640**
**US-A1- 2005 215 640**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This application claims priority to United States Patent Application Serial No. 61/287,857 filed December 18 2009.

### Field of the Invention

The invention relates generally to a more efficient and more effective delivery system for β-Hydroxy-β-methylbutyrate (HMB) and more specifically to administration of HMB free acid resulting in a more efficient and more effective way of administrating HMB over administration of a similar dosage of the calcium salt form of HMB (CaHMB).

### Background of the Invention

HMB has been found to be useful within the context of a variety of applications. Specifically, in U.S. Patent No. 5,360,613 (Nissen), HMB is described as useful for reducing blood levels of total cholesterol and low-density lipoprotein cholesterol. In U.S. Patent No. 5,348,979 (Nissen et al.), HMB is described as useful for promoting nitrogen retention in humans. U.S. Patent No. 5,028,440 (Nissen) discusses the usefulness of HMB to increase lean tissue development in animals. Also, in U.S. Patent No. 4,992,470 (Nissen), HMB is described as effective in enhancing the immune response of mammals. U.S. Patent No. 6,031,000 (Nissen et al.) describes use of HMB and at least one amino acid to treat disease-associated wasting. In U.S. Patent No. 6,103,764, HMB is described as increasing the aerobic capacity of muscle of an animal without a substantial increase in the mass of the muscle. In addition, HMB has been described as useful for improving a human's perception of his emotional state in U.S. Patent No. 6,291,525.

HMB has been shown to have positive effects on maintaining and increasing lean muscle mass in cancer cachexia and AIDS wasting. In addition, a positive effect on muscle damage and the resulting inflammatory response caused by exercising which leads to muscle soreness, strength loss, and an increase in pro-inflammatory cytokines is seen with use of HMB.

It has previously been observed that HMB alone or in combination with other amino acids is an effective supplement for restoring muscle strength and function in young athletes. Further, it has been observed that HMB in combination with two amino acids, arginine and lysine, is effective in increasing muscle mass in elderly persons.

HMB is an active metabolite of the amino acid leucine. The use of HMB to suppress proteolysis originates from the observations that leucine has protein-sparing characteristics (18; 24). The essential amino acid leucine can either be used for protein synthesis or transaminated to the α-ketoacid (α-ketoisocaproate, KIC) (24). In one pathway, HMB is formed in the liver via oxidation of the leucine transamination product α-ketoisocaproate. Approximately 5% of leucine oxidation proceeds via this pathway (28). HMB is superior to leucine in enhancing muscle mass and strength. The optimal effects of HMB can be achieved at 3.0 grams per day, or 0.038g/kg of body weight per day, while those of leucine require over 30.0 grams per day (29).

Once produced or ingested, HMB appears to have two fates. The first fate is simple excretion in urine. After HMB is fed, urine concentrations increase, resulting in an approximate 20-50% loss of HMB to urine (26; 52). Another fate relates to the activation of HMB to HMB-CoA (4; 6; 16; 17; 20; 35; 36; 41; 43; 54). Once converted to HMB-CoA, further metabolism may occur, either dehydration of HMB-CoA to MC-CoA, or a direct conversion of HMB-CoA to HMG-CoA (42), which provides substrates for intracellular cholesterol synthesis. Several studies have shown that HMB is incorporated into the cholesterol synthetic pathway (2-4; 16) and could be a source of cholesterol for new cell membranes that are used for the regeneration of damaged cell membranes (29). Human studies have shown that muscle damage following intense exercise, measured by elevated plasma CPK (creatine phosphokinase), is reduced with HMB supplementation. The protective effect of HMB has been shown to manifest itself for at least three weeks with continued daily use (14; 22; 23).

*In vitro* studies in isolated rat muscle show that HMB is a potent inhibitor of muscle proteolysis (32) especially during periods of stress. These findings have been confirmed in humans; for example, HMB inhibits muscle proteolysis in subjects engaging in resistance training (26). The results have been duplicated in many studies (14; 22; 33; 46; 53) (9-11; 47; 48; 48) In C2C12 muscle cells, HMB attenuates experimentally-induced catabolism (e.g..

The molecular mechanisms by which HMB decreases protein breakdown and increases protein synthesis have been reported (10; 44). In mice bearing the MAC16 cachexia-inducing tumor, HMB attenuated protein degradation through the down-regulation of key activators of the ubiquitin-proteasome pathway (47). Furthermore, HMB attenuated proteolysis-inducing factor (PIF) activation and increased gene expression of the ubiquitin-proteasome pathway in murine myotubes, thereby reducing protein degradation (48). PIF inhibits protein synthesis in murine myotubes by 50% and HMB attenuates this depression in protein synthesis (9). Eley et al demonstrated that HMB increases protein synthesis by a number of mechanisms, including the down-regulation of eukaryotic initiation factor 2 (eIF2) phosphorylation through an effect on dsRNA-dependant protein kinase (PKR) and upregulation of the mammalian target of rapamycin/70-kDa

ribosomal S6 kinase (mTOR/p70$^{s6k}$) pathway. The net result is increased phosphorylation of 4E-binding protein (4E-BP1) and an increase in the active eIF4G.eIF4E complex. Leucine shares many of these mechanisms with HMB, but HMB appears to be more potent in stimulating protein synthesis (9).

HMB can also increase protein synthesis by attenuating the common pathway that mediates the effects of other catabolic factors such as lipopolysaccharide (LPS), tumor necrosis factor- $\alpha$/interferon- y (TNF-$\alpha$ /IFN-$\gamma$), and angiotensin II (Ang II) (10; 11). HMB acts by attenuating the activation of caspases-3 and -8, and the subsequent attenuation of the activation of PKR and reactive oxygen species (ROS) formation via down-regulation of p38 mitogen activated protein kinase (p38MAPK). Increased ROS formation is known to induce protein degradation through the ubiquitin-proteasome pathway. HMB accomplishes this attenuation through the autophosphorylation PKR and the subsequent phosphorylation of eIF2$\alpha$, and in part, through the activation of the mTOR pathway.

A recent report tested the hypothesis that HMB, like leucine, would stimulate mTORc1 independent of PI3K signaling in C2C12 myotubes (19). HMB stimulated the phosphorylation of AKTSer473 (+129%), S6K1Thr389 (+50%) and 4EBP1Thr65/70 (+51%). HMB stimulated anabolic signaling with greater potency than leucine, e.g. S6K1Thr389 +50% vs. +17%; respectively. As expected, incubation of HMB with rapamycin (mTORc1 inhibitor) ablated increases in mTORc1 signaling, but not AKT phosphorylation (+188%). In contrast, incubation with LY290042 (PI3K inhibitor) abolished HMB-induced increases in both AKT and mTORc1 signaling, suggesting HMB signals to mTORc1 in a PI3K-dependent manner. These data suggest that HMB, despite being a leucine metabolite, signals to mTORc1 through mechanisms distinct from those of leucine.

Numerous studies have shown an effective dose of HMB to be 3.0 grams per day as CaHMB (~38 mg/kg body weight-day$^{-1}$). As a dietary supplement, HMB has been used as the mono-hydrated calcium salt, whose empirical formula is $Ca(HMB)_2$-$H_20$. This dosage increases muscle mass and strength gains associated with resistance training, while minimizing muscle damage associated with strenuous exercise (14; 26; 30; 33). HMB has been tested for safety, showing no side effects in healthy young or old adults (15; 25). HMB in combination with L-arginine and L-glutamine has also been shown to be safe when supplemented to AIDS and cancer patients (38).

Studies in humans have also shown that dietary supplementation with 3.0 grams of CaHMB per day plus amino acids attenuates the loss of muscle mass in various conditions such as cancer and AIDS (5; 12). A meta-analysis of supplements to increase lean mass and strength with weight training showed HMB to be one of only 2 dietary supplements that increase lean mass and strength with exercise (30). More recently it was shown that HMB and the amino acids arginine and lysine increased lean mass in a non-exercising, elderly population over a year-long study.

Leucine oxidation increases after exercise, and optimal levels of HMB during and just after exercise would be desired for optimal prevention of muscle damage and subsequent recovery. Further, the inflammatory process is stimulated during an injury, which if left unchecked is deleterious and delays healing. Chronic inflammation and pro-inflammatory cytokines have been shown to be a major underlying and causative factor in cardiovascular disease and type II diabetes, as well as in asthma, autoimmune diseases, inflammatory bowel disease, chronic obstructive pulmonary disease and rheumatoid arthritis.

Human studies have shown a positive effect of resistance exercise on muscle protein synthesis as early as 1-2 hours post exercise and lasting up to 48 hours (8; 34). Studies have also shown that the timing of nutrient availability to be critical for maximal post-exercise stimulation of protein synthesis as well as blunting of protein breakdown (40). The most optimal time for delivery of nutrient appears to be within 2 hours post-exercise. Dreyer at al. (7) demonstrated that the ingestion of a leucine-rich nutrient solution within 1 h of post-exercise recovery resulted in significant enhancement of the mTOR signaling pathway and muscle protein synthesis.

The dissociation curve of CaHMB is identical to that of calcium acetate (49) resulting in peak plasma HMB levels ranging from 60 to 120 minutes after ingestion depending upon the dosage given. Time to peak plasma levels after a typical 1 gram dosage was 2 hours (52), thus requiring CaHMB to be taken before exercise for maximal benefit.

Thus, the timing of HMB administration and the HMB level in the blood are important to the efficacy of HMB on muscle. The need exists for a faster and more efficient delivery system for HMB.

## Summary of the Invention

The invention relates to the administration of HMB in a free acid form ("HMB-acid").
Administration using HMB free acid improves HMB availability to tissues and thus provides a more rapid and efficient method to get HMB to the tissues than administration of CaHMB. The oral intake or sublingual administration of a free acid HMB associated with a matrix results in direct and rapid absorption of HMB, offering an improved method of delivery resulting in an increased availability of HMB to the tissues.

In one example, the HMB free acid is delivered directly by neutralizing HMB free acid in a soluble matrix such as a gel. The HMB free acid is administered orally or sublingually to a person in an effective amount.

Brief Description of the Figures

Figure 1 shows plasma levels of CPK and LDH after a strenuous exercise bout.
Figure 2 shows muscle strength and subjective soreness after a strenuous bout of exercise.
Figure 3 shows plasma HMB levels as found in Experimental Example 1.
Figure 4 shows peak plasma HMB concentration and time to peak concentration.
Figure 5 shows plasma HMB levels.
Figure 6 shows peak plasma HMB concentration and time to peak concentration.
Figure 7 shows percent of HMB dosage excreted in the urine.
Figure 8 shows a treatment regime of the present invention.
Figure 9 shows changes in CPK after an acute bout of eccentric exercise.

Detailed Description of Preferred Embodiments

The invention relates to a method of delivery of HMB to a person, and specifically a method of administering HMB-acid to a person such that the administration of free acid HMB results in an increase in effectiveness of HMB over administration of other forms of HMB, including CaHMB. Use of HMB-acid results in the improvement of HMB availability to a person's tissues. The administration of HMB-acid increases effectiveness for protecting against muscle damage and accompanying inflammatory response over administration of HMB in its other forms. Further, administration of free acid HMB may also preserve muscle in cachectic and wasting conditions and act to blunt inflammation, including chronic inflammation that may cause a number of diseases, such as cardiovascular disease. The unexpected and surprising discovery that free acid HMB decreases muscle damage better than CaHMB indicates that it may also decrease inflammatory response resulting from the damage. The administration of HMB free acid has widespread applications as a nutritional or medical supplement and may affect a large portion of the population.

In accordance with the present invention, HMB is administered to humans in its free acid form. The free acid HMB may be associated with a carrier, such as a matrix or gel. In the preferred embodiment, the free acid HMB is administered orally or sublingually, although any means of administering HMB is appropriate. HMB-acid is commercially available.

HMB in its acid form is called 3-hydroxy-3-methylbutyric acid, β-hydroxy-β-methylbutyric acid, or β-hydroxy-isovalaryic acid and can be designated "HMB-acid." The structural formula is $(CH_3)_2C(OH)CH_2COOH$ and the molecule is:

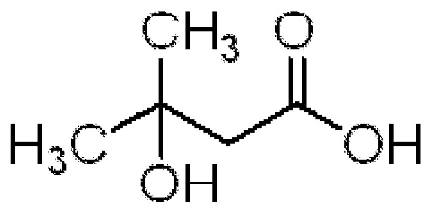

In the present invention, HMB-acid is administered to a human in an effective amount. An effective amount includes a range from about 0.01 grams to about 0.2 grams of HMB-acid per kilogram body weight in twenty-four (24) hours. HMB-acid may also be administered to a human in an effective amount from about 0.5 grams to about 30 grams of HMB-acid per day. An effective amount of HMB-acid will result in a greater increase in plasma levels of HMB and/or will result in a faster time to reach peak plasma levels of HMB relative to administration of a similar dosage of CaHMB The increase in the effectiveness with administration of HMB-acid may be 10%, 20%, 30%, 50%, 75%, 100%, 200%, 400%, 500% or greater than administration of a similar dose of CaHMB. Comparison of HMB-acid with other forms of HMB may be based on effectiveness or efficiency of HMB using standard indices known to those of skill in the art.

In the Examples, the HMB-acid is administered as a soluble gel, although the invention is not limited to use of a soluble gel or matrix with HMB-acid. HMB-acid in any pharmaceutically acceptable form, including but not limited to solids, tablets, capsules, and liquids such as oral intravenous solutions, is within the scope of this invention. The HMB-acid can be administered utilizing any pharmaceutically acceptable carrier, including but not limited to various starches and saline solutions. In the preferred embodiment, an effective amount of HMB-acid is administered as two or three daily doses, although a single dose of an effective amount of HMB-acid per day will be understood to be within the scope of the invention, as would be any other number of doses of HMB during the day.

The delivery of HMB-acid, most typically as a HMB-acid soluble matrix such as a gel, results in a significant improvement in HMB's anabolic effect with marked reductions in CPK over administration of HMB as a salt, including

CaHMB or administration of HMB in other forms such as an ester or lactone. In one embodiment, the administration of HMB-acid gel results in a doubling of the plasma peak of HMB in about ¼ of the time as administration of a similar dosage of CaHMB, and has a 25% improved efficiency of delivery as measured by plasma clearance over a similar dosage of CaHMB.

This method of delivery has widespread applications. Known uses or benefits of HMB include, but are not limited to, improved nitrogen retention and protein sparing, improving lean body mass, improving muscle function and/or muscle performance, decreasing muscle damage in muscle subjected to stress or damage, decreasing inflammatory response after muscle is subjected to stress or damage, improving the body's immune response after stress or damage, treating disease associated wasting (such as wasting associated with cancer, chronic pulmonary disease, age, chronic kidney disease, long-term hospitalization or AIDS), improving a lipid profile such a low density lipoprotein (LDL) to high density lipoprotein (HDL), and improving a person's emotional state. A more effective and more efficient way to administer HMB has widespread applications in all of these known uses of HMB.

While use of HMB-acid has previously been stated, HMB in free acid form was thought to be equivalent to HMB in the calcium salt and other salts as proposed administration forms in the prior art. Differences in the effectiveness of HMB-acid and HMB salts were not previously tested.

Previously, numerous obstacles existed to both extensive testing and commercial utilization of the free acid form of HMB, and since it was thought there was no difference, the calcium salt was adopted as a commercial source of HMB. Until recently packaging and, in particular, distribution of dietary supplements has been better suited to handle nutrients in a powdered form and therefore the calcium salt of HMB was widely accepted. HMB-acid is a liquid and much more difficult to deliver or incorporate into products.

Unlike other calcium salts, it had been shown that the calcium and HMB components of the molecule dissociate very easily, therefore, adding to the assumption that there would be no physiological difference between HMB free acid and the calcium salt of HMB (19)..(49).

Additionally, formation and crystallization of the calcium salt of HMB had been utilized as a final purification stage in the manufacturing process. One compound in particular that the crystallization served to limit was 3,3-Dimethyl acrylic acid. This compound adds a very off flavor which is hard to mask. Currently, the manufacturing process for HMB has allowed for HMB free acid to be produced in a purity that allows for oral ingestion of the HMB free acid. Besides currently having a commercial source that is pure enough for oral ingestion, the HMB- acid needs to be buffered for oral ingestion, a process which only recently was determined due to the factors listed above which precluded previous use of HMB-acid.

Because the calcium and HMB in the calcium salt was loosely associated (49), it was previously thought that there would be no difference in oral administration of HMB either as a free acid or as a calcium salt (19). As shown in Example 1, not only is there a surprising difference in plasma levels attained with oral administration of HMB-acid, but there is a 25% increase in plasma clearance which indicates increased utilization of HMB by tissues with resultant improved effects on muscle mass and function. When given in molar equivalents, HMB in free acid form results in double the plasma level of HMB in about one fourth (¼) the time when compared to the calcium salt of HMB. The improved effect on muscle is clearly shown in Example 2 in that HMB administered in free acid form is more protective than CaHMB when muscle is subjected to acute exercise.

While it is known that CaHMB is soluble in and easily dissociates in aqueous solutions of neutral to acidic pH, one of skill in the art would predict a short lag time in appearance due to this dissociation step. CaHMB administered in a gelatin capsule has a dissolution time of between 10 and 15 minutes in the gut. Therefore, one skilled in the art would expect similar absorption and thus peak plasma levels of HMB whether the HMB was administered as CaHMB or as HMB-acid, however, slightly delayed for the differences described. Contrary to these expectations, however, it was found that there was a significant difference in plasma peak between the two forms. In some instances, the difference in peak plasma times was ninety (90) minutes, which is several fold longer than should be accounted for by capsule dissolution and disassociation of the CaHMB.

One skilled in the art would also predict similar plasma peaks and areas under the curve since a similar amount of the nutrient HMB was being released into the gut with the CaHMB curve shifted further out in time. An unexpected result of administering HMB-acid is the doubling in peak plasma HMB levels.

An additional unexpected result is the greater clearance (utilization) of HMB once in the plasma. Many nutrients have similar plasma and urinary profiles unless there is a conservation mechanism in the kidney for that nutrient. None is presently known for HMB and thus one skilled in the art might presume a much higher percentage of the dosage would be excreted in the urine with the doubling of the plasma peak in HMB concentrations. Again, this was not observed and is an unexpected finding. This coupled with the higher clearance rate show improved utilization of HMB by tissues which again was a surprising finding.

As shown in the Experimental Examples, and specifically in Example 2, administering HMB-acid is more quickly effective in minimizing muscle damage after exercise than CaHMB. This second example shows a benefit that could not have been predicted directly from the findings of Example 1.

The method of this invention is further illustrated by the following experimental examples.

EXPERIMENTAL

Example 1: Absorption of HMB-Acid Gel Compared with CaHMB in Capsule Form

Materials and Methods

***Human subjects.*** In study 1, four male and four female college-aged subjects were studied. In study 2, an additional four male and four female college-aged subjects were studied. The protocols for both studies were approved by the Iowa State University IRB and each subject gave informed consent to participate in the study. Due to the nature of the treatments, neither the subjects nor the researchers could be blinded.

***Treatments.*** The same treatments were given to the subjects in both study groups. The three treatments were given in random order to each subject with at least a one week washout period between treatments. The treatments were supplied by Metabolic Technologies, Inc. (MTI, Ames, IA) and were prepared with food grade ingredients. One gram of CaHMB or the equivalent HMB in free acid in a gel form was administered to the subjects. The CaHMB capsules were obtained from a commercial supplement manufacturer (Optimum Nutrition, Aurora, IL) while the HMB- acid gel was prepared at MTI laboratories. Briefly, the HMB-acid was adjusted to a pH 4.5 with potassium carbonate ($K_2CO_3$) and flavors and sweeteners were then added. The 1.0 g CaHMB capsule was taken with 355 mL of water (approx. 12 oz.). The free acid gel dosage was 0.80 g and was equivalent to the free acid contained in the CaHMB in the capsule. The free acid gel treatments were either swallowed (FASW) or held sublingual for 15 seconds and then swallowed (FASL). FASW consisted of expelling the entire dose into the mouth in a 3 ml syringe, swallowing, and then following this with 355 mL of water. FASL subjects were instructed to place the entire dosage under the tongue and hold the dosage for 15 sec before swallowing. They then rinsed and followed the dose with 355 mL of water.

***Study 1 design.*** For study 1 the subjects reported to the laboratory in the morning following an overnight fast. Before ingestion of one of the supplemental treatments, a flexible sterile polyethylene catheter was inserted into a forearm vein using sterile procedures and a pre-ingestion blood sample was drawn. Subsequent blood samples were taken at 0, 2, 5, 10, 15, 25, 35, 45, 60, 90, 120 and 180 min after ingestion of the treatment. Plasma was separated and samples were stored frozen at -70° C for analysis of HMB concentration. In addition, a portion of the pre-ingestion and 180 minute blood samples were used for measurements (LabCorp, Kansas City, MO) of glucose, uric acid, blood urea nitrogen (BUN), creatinine, sodium, potassium, chloride, carbon dioxide, phosphorous, protein, albumin, globulin, albumin:globulin ratio, total bilirubin, direct bilirubin, alkaline phosphatase, lactate dehydrogenase, aspartate aminotransferase (AST), alanine aminotransferase (ALT), gamma-glutamyl transpeptidase (GGT), iron binding capacity (TIBC), unsaturated iron binding capacity (UIBC), iron, iron saturation, total cholesterol, triglycerides, high density lipoprotein (HDL), low density lipoprotein (LDL), and cholesterol ratio. A complete blood count (CBC) was also performed before and after the 180 min treatment period. Subjects also completed a brief questionnaire to report any physical symptoms (such as nausea, headache, etc) they may have experienced during the experiment.

***Study 2 design.*** Study 2 was conducted similar to study 1 with the following modifications. In study 2, plasma levels of HMB were measured for 1440 min (24 h) and total urine collection was also performed for measurement of urinary HMB excretion during this period. After the 180 min blood sample, the subjects were allowed to leave the laboratory and were instructed to return to the laboratory for additional blood samples at 360, 720, and 1440 min after the ingestion of the supplement. As in study 1, each subject took each of the treatments with at least a one week washout period between treatments. Samples were again stored frozen at -70° C for analysis of plasma and urinary HMB concentrations. Pre-ingestion, 180 min and 1440 min blood samples were again assayed for the same measurements already for study 1. Subjects were provided with a standardized lunch after the 180 min blood sampling and instructed to eat this at approximately 240 min post ingestion. Following the 720 min blood sample, subjects were instructed to eat a normal evening meal before 10 pm. Subjects reported back to the laboratory the following morning for the fasted 1440 min blood sample. A urine collection container was provided and subjects collected all urine produced during the experimental 24 h experimental period. The urine was stored refrigerated when not being collected. Urine volumes were measured and samples of the total urine collection were taken and stored frozen at -70°C until analyzed for HMB.

***HMB analysis.*** Plasma and urine HMB were analyzed by gas chromatography/mass spectrometry (GC/MS) as previously described.(27)

***Calculations and statistics.*** Areas under the curves were calculated for each subject using the trapezoidal method that sums the area above baseline.(51) Half-life of plasma HMB was calculated for study 2. The following equations were used:

$$k=(\ln(C_{peak})-\ln(C_{trough}))/T_{interval}$$

$$t_{1/2}=0.693/k$$

Peak plasma concentrations for each subject were used for $C_{peak}$. Trough concentrations, $C_{trough}$ were the concentrations measured at 720 min, because the 720 min plasma concentrations were not significantly different from baseline. $T_{peak}$ was the time at which $C_{peak}$ was measured and $T_{interval}$ is the time from $T_{peak}$ until the time at $C_{trough}$ (720 min). The extracellular fluid compartment was assumed to be 20% of body weight and calculated using equation 3 below(1). The plasma clearance of HMB was then calculated by multiplying the extracellular fluid compartment, $V_d$, by the elimination constant, $K_{el}$, as shown in equation 4{Thalhammer, 1998 9588 /id}.

$$V_d = \text{Body wt}(0.20)$$

$$\text{Clearance} = V_d(K_{el})(50).$$

The data were analyzed using a crossover design with Proc GLM in SAS. (45) For the timed sampling of plasma HMB a repeated measures polynomial model was used. The model included subject, order, and treatment main effects and time by treatment interaction where appropriate. For other parameters Proc GLM was also used with subject, order, and treatment main effects and the p values are reported for the treatment main effect. Statistical significance was determined for $p<0.05$ and a trend was determined for $0.05<p<0.10$.

**Results**

The results of the studies are shown in Figs. 1-7. Figure 1 shows plasma levels of CPK and LDH after a strenuous exercise bout. Figure 2 shows muscle strength and subjective soreness after a strenuous bout of exercise. Figure 3 shows plasma HMB levels in study 1. Values are means +/-SEM, n=4 males and 4 females. *=p,0.05, +=p,0.001, +p,0.0001 for free acid treatments versus CaHMB capsules. Figure 4 shows Study 1 peak plasma HMB concentration and time to peak concentration after a single dose of either CaHMB, HMB-acid gel swallowed (FASW, or HMB-acid gel held sublingual and then swallowed (FASL). **=p,0.0002. Figure 5 shows plasma HMB levels in sugdy 2. Values are means +/-SEM, n = 4 males and 4 females. *=p,0.05, +=p,0.01, ++p,0.0001. Figure 6 shows Study 2 peak plasma HMB concentration and time to peak concentration after a single dose of either CaHMB, HMB free acid gel swallowed (FASW, or HMB free acid gel held sublingual then swallowed (FASL). **=p<0.0002 for concentration and p<0.0001 for time. Figure 7 shows percent of the HMB dosage excreted in the urine over 24 h post ingestion and relative percentage of the dose retained. *=p<0.002.

***Subject characteristics.*** Table 1 shows the subject characteristics for studies 1 and 2. Each study was balanced for gender and each treatment group maintained steady weights throughout the 3 testing periods. In each study all subjects completed all 3 testing protocols. No adverse treatment effects, such as nausea after taking the treatments, were reported in either study.

***Study 1 results (Tables 2-4).*** Table 2 illustrates the peak plasma concentrations ($C_{peak}$) and time to reach peak plasma concentrations ($_tp_{eak}$). CaHMB taken by capsule resulted in a peak plasma HMB level of 131±10 nmol/mL, whereas HMB taken either by free acid gel FASW or FASL delivery resulted in significantly greater (259±24 and 231±21 nmol/mL, respectively, p<0.0001) and earlier (33.1±4.6 and 36.3±1.3 min, respectively) peaks in plasma HMB levels compared with CaHMB by capsule at 121.9±15.6 h (p<0.0001). At 180 min, plasma HMB for all delivery methods was still elevated above baseline, and there were no treatment differences among the three groups. Consequently, Areas under the curve (AUC) for plasma HMB levels following the 3 treatments are also shown in Table 2. HMB administered in free acid gel form resulted in 97% and 91% greater areas under the curve (AUC) for FASW and FASL, respectively (p<0.0001). There were no differences between HMB-acid gel delivered by FASW or by FASL.

Table 3 shows blood chemistries for study 1. There were no significant main effect treatment differences for any of the measured time points or differences. Table 4 shows blood hematology measured in study 1. The FASW group had significantly greater decrease in absolute lymphocyte numbers over the measurement period (p<0.04) due primarily to the fact that the FASW group tended to have higher lymphocyte numbers at the start of the study (p<0.09). There were no significant differences in lymphocyte numbers at the end of the study and all the means were within normal

limits for lymphocyte number.

***Study 2 results (Tables 2, 5-7).*** Study 2 was conducted to look at plasma HMB for a 24 hour period as well as to measure urinary losses during this same time period. Similar to study 1, a rapid increase in and significantly greater plasma HMB with HMB given in free acid gel form for both FASW and FASL than with CaHMB in capsule form was seen. At 180 min all treatments resulted in similar plasma HMB levels (approximately 110 nmol/mL). CaHMB by capsule did maintain a slightly higher plasma HMB level at 360 and 720 min ($p<0.05$).

Table 5 lists peak plasma HMB concentrations ($C_{peak}$), time to peak plasma HMB concentrations ($t_{peak}$), and plasma HMB half-life. Plasma HMB level for CaHMB by capsule peaked at 131.2±6.0 nmol/mL at 135.0±17.0 min, while FASW and FASL resulted in greater HMB plasma peaks ($p<0.0003$) in shorter time ($p<0.0001$), 238.6±16.0 nmol/mL at 41.9±5.8 min and 247.6±19.8 nmol/mL at 38.8±2.6 min for FASW and FASL, respectively.

Plasma half-life as shown in Table 5 for CaHMB by capsule was 3.17±0.22 h. Half-lives for HMB FASW and HMB FASL were 2.50±0.13 and 2.51±0.14 h, respectively ($p<0.004$). Area under the curve and urinary HMB measured in study 2 are also shown in Table 5. Over the 24 hours period AUC for HMB administered as the free acid gel was significantly greater by 15.4 and 14.3% for FASW and FASL , respectively, than for CaHMB administered in capsules ($p<0.001$). Despite the significant increase in peak plasma HMB and AUC, urinary HMB losses were not significantly greater for the free acid gel treatments; urinary HMB losses were 14.7±2.0, 17.8±2.9, and 17.2±2.5% of the initial dosage lost for CaHMB, FASW and FASL, respectively. There was an approximately 25% increase in HMB clearance with the free acid gel form compared with the CaHMB form ($P < 0.003$).

Blood chemistries measured during study 2 are shown in Table 6 and blood hematology measured in study 2 is shown in Table 7. Statistical analysis of the change in parameters over the 24 h measurement period showed no significant statistical changes ($p<0.05$) for any of the chemistry or hematology values measured. There was a strong trend for a difference with chloride ($p<0.06$). FASL showed a greater increase over the period compared with FASW; however neither FASW nor FASL was significantly different from CaHMB. A trend was also seen with sodium where FASW showed a larger increase over the measurement period than FASW or CaHMB ($p<0.07$). Means for chloride and sodium over the study period were well within normal values.

**Discussion**

It is clear from the present studies that oral or sublingual administration of HMB in free acid gel form resulted in more rapid and sustained increases in plasma HMB than when compared HMB administered as the calcium salt (CaHMB) in a hard gelatin capsule. Free acid gel administration of HMB resulted in significant increases (average +14.8%) in the AUC for plasma HMB without any major changes in plasma HMB half-lives or urinary losses. Combined this resulted in significantly increased clearance of HMB and utilization by tissues that was 25% greater than that of the CaHMB form. The data show the improved efficiency of this form of delivery of HMB.

The findings from this study related to plasma kinetics of CaHMB delivery agree with those previously reported by Vukovich et al (52) There is congruence of data related to peak plasma levels, time to peak and plasma half lives, occurred despite significant differences in mode of delivery of CaHMB (four 250 mg capsules by Vukovich et al vs. 1 gram capsule in the present studies). Interestingly, the delivery of HMB as free acid in a gel form resulted in faster peak levels (nearly 90 minutes earlier with the use of the free acid form) but similar half lives, despite plasma levels reaching almost 2-fold those achieved with oral administration of CaHMB. Several studies have supported the use of HMB as a nutritional supplement during exercise. HMB has been shown to decrease muscle protein and membrane breakdown (22; 23; 26) and to enhance protein synthesis. (10) It would therefore be advantageous to have high levels of plasma HMB during the exercise period, and to have HMB retention to be as good as or even better than those previously reported (26; 29; 52). In this regard, oral administration of CaHMB would need to be administered at least 2 hours before any serious stressful bout of exercise, whereas HMB free acid gel may be administered before the exercise bout and have an almost immediate effect.

Delivery of the free acid form of HMB was also associated with significantly higher retention of HMB. Administration of the free acid gel form resulted in a significant increase in AUC while not significantly increasing urinary excretion which would indicate more HMB retention and utilization by the tissues compared with the CaHMB form. The estimated amount of HMB retained was 25% greater with the HMB-acid gel compared with the CaHMB form based upon plasma clearance. Previous studies by Vukovich et al showed that the oral delivery of 3 grams of CaHMB resulted in plasma peak levels that were 3 times higher than those achieved with a 1 gram dose (52). Nissen et al demonstrated a dose-dependent response to oral administration of CaHMB given twice daily at either 1.5 or 3 grams per day. They demonstrated the optimal dose of CaHMB to be 3.0 grams per day resulting in decreased release of creatine kinase (CK), an indicator of muscle damage, and 3-methylhistine, an indicator of protein breakdown (26). Similar findings were reported by Gallagher who also showed that daily administration of 6 grams per day would be more beneficial than 3 grams per day (14). Taken together, the above studies indicate a benefit to having more HMB available to the muscles during exercise.

HMB delivery by free acid gel results in a faster and greater peak in HMB blood levels as well as equally sustained levels when compared with CaHMB administered in a capsule. This form of delivery is equally safe as those currently and previously (15; 25) found with oral administration of CaHMB.

**Table 1. Subject Descriptors.**

| | Treatment | | |
|---|---|---|---|
| | **CaHMB Capsule** | **HMB-Acid Gel Swallow** | **HMB-Acid Gel Sublingual** |
| | | **Study 1** | |
| Gender, male/female | 4/4 | 4/4 | 4/4 |
| Age, y Body Weight, kg | 23.8±1.3 | 23.8±1.3 | 23.8±1.3 |
| All | 68.5±3.9 | 68.5±3.8 | 68.6±4.0 |
| Females | 63.2±4.5 | 63.5±4.5 | 63.5±4.9 |
| Males | 73.7±5.6 | 73.5±5.6 | 73.8±5.9 |
| | | **Study 2** | |
| Gender, male/female | 4/4 | 4/4 | 4/4 |
| Age, y Body Weight, kg | 22.4±1.0 | 22.4±1.0 | 22.4±1.0 |
| All | 72.0±4.2 | 72.4±4.1 | 72.3±4.1 |
| Females | 66.8±3.2 | 67.4±3.3 | 67.0±3.5 |
| Males | 77.2±7.2 | 77.4±7.2 | 77.7±6.9 |

**Table 2. Study 1 Area Under the Curve, Concentration peak ($C_{peak}$) and Time to Reach Concentration Peak ($t_{peak}$). Study 1 Treatment[1]**

| | **CaHMB** | **Free Acid Gel Swallow** | **Free Acid Gel Sublingual** | **p Value[2]** |
|---|---|---|---|---|
| $C_{peak}$, nmol/mL | 131.2±10.1 | 259.1±23.9 | 231.2±21.0 | 0.0001 |
| $t_{peak}$, min | 121.9±15.6 | 33.1±4.6 | 36.3±1.3 | 0.0001 |
| AUC, nmol/180 min | 13,997±1,534 | 27,532±1,742 | 26,778±1,980 | 0.0001 |

[1]Mean±SEM.
[2]P value for treatment differences.

**Table 3.Blood Chemistry Studyl.[1]**

| | CaHMB | | | Free Acid Gel Swallow | | | Free Acid Gel Sublingual | | | p Value[2] |
|---|---|---|---|---|---|---|---|---|---|---|
| | Before | After | % Change | | After | % Change | Before | After | % Change | |
| Glucose, mg/dL | 72.6 ±1.6 | 76.4 ± 3.2 ± | **5.2%** | 70.5±1.7 | 73.3±4.1 | **3.9%** | 74.1±1.7 | 77.4±2.5 | **4.4%** | **0.83** |
| Uric Acid, mg/dL | 4.5±0.3 | 4.5±0.4 | **-1.4%** | 4.9±0.4 | 4.9±0.4 | **0.8%** | 4.0±0.4 | 4.0±0.5 | **-2.2%** | **0.47** |
| Blood Urea Nitrogen, mg/dL | 12.1±1.0 | 11.3+0.9 | **-7.2 %** | 15.4±1.6 | 14.4±1.5 | **-6.5%** | 14.4±0.8 | 12.9±0.8 | **-10.4%** | **0.25** |
| Creatinine, mg/dL | 0.9±0.1 | 0.9±0.1 | **0.5%** | 1.0±0.1 | 0.9±0.1 | **-4.7%** | 1.0±0.1 | 1.0±0.04 | **-1.5 %** | **0.41** |
| Sodium, mEq/L | 139.1±0.5 | 139.4±0.8 | **0.2%** | 138.5±1.1 | 138.3±0.9 | **-0.2%** | 139.3±0.8 | 137.8±0.9 | **-1.1 %** | **0.25** |
| Potassium, mEq/L | 4.1±0.1 | 4.7±0.2 | **13.4%** | 4.2±0.1 | 4.6±0.3 | **11.4%** | 4.0±0.03 | 4.5±0.1 | **10.6%** | **0.98** |
| Chloride, mEq/L | 103.3±0.6 | 103.1±0.7 | **-0.1%** | 103.6±0.8 | 103.3±0.5 | **-0.4%** | 102.9±0.6 | 102.4±1.1 | **-0.5%** | **0.98** |
| CO2, mEq/L | 23.4±0.6 | 24.8±0.5 | **5.9%** | 22.4±0.7 | 24.3±0.5 | **8.1%** | 24.0±0.5 | 24.3±0.6 | **1.0%** | **0.17** |
| Phosphorus. mg/dL | 3.9±0.2 | 3.5±0.3 | **-10.8%** | 4.0±0.1 | 3.4±0.1 | **-15.6%** | 4.1±0.05 | 3.5±0.2 | **-13.2%** | **0.71** |
| Protein, g/dL | 6.3±0.2 | 6.5±0.3 | **3.2 %** | 6.2±0.2 | 6.3±0.2 | **1.4%** | 6.3±0.2 | 6.4±0.2 | **1.6%** | **0.77** |
| Albumin, g/dL | 4.0±0.1 | 4.1±0.1 | **0.3%** | 4.0±0.2 | 4.1±0.2 | **1.9%** | 4.1±0.1 | 4.1±0.2 | **1.5%** | **0.77** |
| Globulin, g/dL | 2.3±0.2 | 2.5±0.2 | **8.2%** | 2.2±0.1 | 2.2±0.1 | **0.6%** | 2.3±0.2 | 2.3±0.1 | **1.6%** | **0.40** |
| A:G Ratio[2] | 1.8±0.1 | 1.7±0.1 | **-8.9%** | 1.9±0.1 | 1.9±0.1 | **0.0%** | 1.9±0.1 | 1.8±0.1 | **-2.0%** | **0.46** |
| Total Bilirubin, mg/dL | 0.5±0.07 | 0.5±0.08 | **7.9 %** | 0.4±0.05 | 0.5±0.07 | **23.3%** | 0.4±0.05 | 0.5±0.05 | **22.6%** | **0.27** |
| Direct Bilirubin, mg/dL | 0.1±0.02 | 0.1±0.01 | **5.8%** | 0.1±0.01 | 0.1±0.01 | **7.9%** | 0.1±0.01 | 0.1±0.01 | **8.6%** | **0.96** |
| Alkaline Phosphatase, IU/L | 66.5±6.5 | 68.4±8.4 | **2.8%** | 63.9±5.8 | 64.4±6.7 | **0.8%** | 66.0±6.3 | 66.8±6.8 | **1.1%** | **0.80** |
| Lactate Dehydrogenase, IU/L | 160.9±9.5 | 165.6±10.7 | **3.0%** | 168.8±10.4 | 166.3±11.1 | **-1.5 %** | 164.8±12.5 | 167.3±12.2 | **1.5%** | **0.87** |
| Aspartate Aminotransferase, IU/L | 21.4±1.5 | 22.4±1.8 | **4.7 %** | 24.0±2.2 | 23.1±2.0 | **-3.6%** | 28.0±7.9 | 28.3±8.2 | **0.9%** | **0.15** |
| Alanine Aminotransferase, IU/L | 14.1±1.5 | 15.6±2.0 | **10.6%** | 15.9±1.5 | 15.6±1.3 | **-1.6%** | 17.4±2.7 | 17.3±2.7 | **-0.7%** | **0.25** |
| Gamma-glutamyl | 13.8±0.9 | 13.1±0.9 | **-4.5 %** | 14.4±1.6 | 13.5±1.1 | **-6.1 %** | 13.3±1.2 | 13.4±1.2 | **0.9%** | **0.45** |
| Iron Binding Capacity, μg/dL | 330.4±32.9 | 342.8±35.9 | **3.7 %** | 328.6±31.0 | 333.4±35.2 | **1.4%** | 328.0±32.8 | 339.5±30.8 | **3.5%** | **0.79** |
| UIBC, μg/dL | 247.1±45.0 | 249.8±47.7 | **1.1%** | 243.1±47.4 | 237.1±49.2 | **-2.5%** | 244.8±42.3 | 244.9±39.5 | **0.1%** | **0.83** |
| Iron, μg/dL | 83.3±16.7 | 93.0±16.8 | **11.7%** | 85.5±19.2 | 96.3±17.3 | **12.6%** | 83.3±16.4 | 94.6±16.6 | **13.7%** | **0.79** |
| Iron Saturation, % | 29.9±9.5 | 31.1±8.9 | **4.2 %** | 30.6±9.4 | 33.5±8.4 | **9.4%** | 29.1±9.0 | 30.9±8.5 | **6.0%** | **0.53** |
| Total Cholesterol, mg/dL | 153.4±9.1 | 153.9±9.7 | **0.3%** | 153.5±5.3 | 153.3±5.6 | **-0.2%** | 154.4±6.9 | 155.5±5.8 | **0.7%** | **0.90** |
| Triglycerides, mg/dL | 80.4±11.6 | 71.6±11.2 | **-10.9%** | 91.3±17.0 | 87.5±14.8 | **-4.1%** | 91.8±16.1 | 83.8±15.8 | **-8.7%** | **0.62** |
| HDL, mg/dL | 51.3±3.2 | 53.1±3.8 | **3.7 %** | 46.9±3.4 | 48.5±3.7 | **3.5%** | 48.6±4.6 | 49.3±4.3 | **1.3%** | **0.69** |
| LDL, mg/dL | 86.1±5.3 | 86.5±5.7 | **0.4%** | 88.4±3.0 | 85.6±4.1 | **-3.1 %** | 87.5±5.7 | 89.4±4.9 | **2.1%** | **0.48** |

(continued)

| | CaHMB | | | Free Acid Gel Swallow | | | Free Acid Gel Sublingual | | | p Value[2] |
|---|---|---|---|---|---|---|---|---|---|---|
| | Before | After | % Change | | After | % Change | Before | After | % Change | |
| Cholesterol Ratio | 3.0±0.1 | 3.0±0.1 | **-2.5 %** | 3.4±0.2 | 3.3±0.3 | **-3.3%** | 3.3±0.2 | 3.3±0.2 | **-0.4%** | **0.72** |

[1]Mean±Standard Error of the Mean.
[2]P value for treatment effect of the difference in starting and ending values indicated by % change for each treatment.

**Table 4. Hematology Values Study 1.[1]**

| | CaHMB | | | HMB-Acid Gel Swallow | | | HMB-Acid Gel Sublingual | | | p Value[2] |
|---|---|---|---|---|---|---|---|---|---|---|
| | Before | After | % Change | Before | After | % Change | Before | After | % Change | |
| WBC, $x10^3/\mu L$ | 6.4±0.4 | 6.4±0.7 | **0.3%** | 6.7±0.5 | 6.1±0.3 | **-10.2%** | 6.6±0.4 | 6.3±0.4 | **-4.2%** | **0.39** |
| RBC, $x10^3/\mu L$ | 4.3±0.2 | 4.3±0.2 | **1.0%** | 4.2±0.2 | 4.2±0.2 | **0.1%** | 4.2±0.2 | 4.3±0.2 | **1.7%** | **0.51** |
| Hemoglobin, g/dL | 13.3±0.4 | 13.4±0.5 | **1.2%** | 13.1±0.5 | 13.0±0.5 | **-0.4%** | 13.1±0.6 | 13.3±0.6 | **1.4%** | **0.54** |
| Hematocrit, % | 38.3±1.1 | 38.7±1.3 | **1.2%** | 37.6±1.6 | 37.6±1.5 | **-0.1%** | 37.8±1.6 | 38.5±1.7 | **2.0%** | **0.44** |
| MCV, fL | 90.0±1.5 | 90.4±1.5 | **0.4%** | 90.1±1.3 | 90.0±1.5 | **-0.1%** | 89.6±1.6 | 89.9±1.7 | **0.3%** | **0.57** |
| MCH, pg | 31.2±0.6 | 31.3±0.6 | **0.3%** | 31.2±0.7 | 31.4±0.8 | **0.7%** | 31.0±0.5 | 31.0±0.5 | **0.0%** | **0.76** |
| MCHC, g/dL | 34.7±0.1 | 34.7±0.2 | **0.1%** | 34.7±0.3 | 34.6±0.3 | **-0.3%** | 34.7±0.2 | 34.5±0.2 | **-0.5%** | **0.64** |
| RDW, % | 13.1±0.2 | 13.1±0.2 | **0.1%** | 13.2±0.2 | 13.2±0.3 | **0.4%** | 13.2±0.2 | 13.3±0.2 | **0.9%** | **0.73** |
| Platelets, $x10^3/\mu L$ | 209.0±14 | 202.9±16 | **-2.9%** | 195.0±31.5 | 202.4±25. | **3.8%** | 221.3±20.8 | 213.9±19.1 | **-3.3%** | **0.31** |
| Neutrophils, % | 50.3±3.3 | 56.1±3.8 | **11.7%** | 46.0±2.8 | 52.9±2.2 | **14.9%** | 47.4±2.2 | 55.4±2.9 | **16.9%** | **0.78** |
| Lymphocytes, % | 39.6±2.8 | 34.0±3.4 | **-14.2%** | 43.8±2.8 | 37.4±1.9 | **-14.6%** | 43.0±1.4 | 35.5±2.1 | **-17.4%** | **0.55** |
| Monocytes, % | 7.1±0.9 | 7.4±0.7 | **3.5%** | 7.6±0.7 | 7.0±0.6 | **-8.2%** | 7.0±1.0 | 7.1±0.7 | **1.8%** | **0.85** |
| Eosinophils, % | 2.5±0.4 | 2.1±0.6 | **-15.0%** | 2.5±0.3 | 2.4±0.4 | **-5.0%** | 2.4±0.4 | 1.8±0.3 | **-26.3%** | **0.41** |
| Basophils, % | 0.5±0.2 | 0.4±0.2 | **-25.0%** | 0.1±0.1[a] | 0.4±0.2 | **200.0%** | 0.3±0.2 | 0.3±0.2 | **0.0%** | **0.14** |
| Neutrophils, $x10^3/\mu L$ | 3.3±0.4 | 3.8±0.7 | **14.5%** | 3.0±0.2 | 3.3±0.2 | **10.9%** | 3.1±0.3 | 3.5±0.3 | **11.6%** | **0.85** |
| Lymphocytes, $x10^3/\mu L$ | 2.5±0.1 | 2.1±0.2 | **-15.6%** | 3.1±0.3 | 2.2±0.1 | **-29.1%** | 2.8±0.2 | 2.2±0.2 | **-22.1 %** | **0.04** |
| Monocytes, $x10^3/\mu L$ | 0.5±0.1 | 0.5±0.1 | **8.3%** | 0.5±0.1 | 0.5±0.04 | **-7.7%** | 0.5±0.1 | 0.5±0.05 | **0.0%** | **0.98** |
| Eosinophils, x103/μL | 0.2±0.04 | 0.1±0.04 | **-21.4%** | 0.2±0.04 | 0.1±0.02 | **-41.2%** | 0.2±0.03 | 0.1±0.02 | **-28.6%** | **0.47** |
| Basophils, $x10^3/\mu L$ | 0.05±0.0 | 0.04±0.0 | **-25.0%** | 0.01±0.01 [a] | 0.04±0.02 | **200.0%** | 0.03±0.02 | 0.03±0.02 | **0.0%** | **0.14** |

[1]Mean±Standard Error of the Mean.
[2]P value for treatment effect of the difference in starting and ending values indicated by % change for each treatment.

**Table 5. Study 2 Plasma HMB Area Under the Curve, Concentration Peak ($C_{peak}$), Time to Concentration Peak ($t_{peak}$), Half-life, Urinary HMB Loss, and HMB Retention.**

| | Study 2 Treatment[1] | | | |
|---|---|---|---|---|
| | **CaHMB** | **HMB-Acid Gel Swallow** | **HMB-Acid Gel Sublingual** | **p Value[2]** |
| $C_{peak}$. nmol/mL | 131.2±6.0 | 238.6±16.0 | 247.6±19.8 | 0.0003 |
| $t_{peak}$, h | 135.0±17.0 | 41.9±5.8 | 38.8±2.6 | 0.0001 |
| Half-life, h | 3.17±0.22 | 2.50±0.13 | 2.51±0.14 | 0.004 |
| AUC, nmol/1440 min | 46,281±2,717 | 53,395±2,862 | 52,886±2,729 | 0.001 |
| 24 h Urine HMB, mmol | 1.00±0.13 | 1.21±0.19 | 1.16±0.17 | 0.18 |
| 24 h Urine HMB, % initial dose | 14.7±2.0 | 17.8±2.9 | 17.2±2.5 | 0.18 |
| Clearance (mL/min) | 53.9±4.2 | 67.3±3.2 | 66.9±1.6 | 0.003 |

[1]Mean±SEM.
[2]P value for treatment differences.
[3]At $C_{peak}$ total extracellular HMB was estimated using 20% body weight as the extracellular volume. It was assumed for this calculation the plasma and extracellular HMB concentrations were equalized.

Amount retained was calculated by subtracting total amount of HMB excreted in the urine. Relative retention percentage indicated in the table should remain unchanged even if this assumption is not met.

**Table 6. Blood Chemistry Study 2.[1]**

| | CaHMB | | | HMB-Acid Gel Swallow | | | HMB-Acid Gel Sublingual | | | p Value[2] |
|---|---|---|---|---|---|---|---|---|---|---|
| | Before | After | % Change | Before | After | % Change | Before | After | % Change | |
| Glucose, mg/dL | 70.8±0.8 | 80.3±2.4 | **13.4%** | 74.1±2.0 | 79.9±1.9 | **7.8%** | 73.0±1.8 | 80.8±2.1 | **10.6%** | **0.62** |
| Uric Acid, mg/dL | 4.8±0.3 | 4.2±0.5 | **-12.0%** | 4.7±0.1 | 4.8±0.3 | **1.9%** | 4.7±0.3 | 4.7±0.3 | **0.8%** | **0.50** |
| Blood Urea Nitrogen, mg/dL | 14.8±1.4 | 13.4±1.0 | **-9.3%** | 14.5±1.4 | 13.9±1.4 | **-4.3%** | 15.3±1.7 | 15.4±1.1 | **0.8%** | **0.55** |
| Creatinine, mg/dL | 1.0±0.1 | 1.0±0.1 | **-1.5 %** | 1.0±0.1 | 1.0±0.1 | **-0.6%** | 1.0±0.1 | 1.0±0.1 | **7.9%** | **0.12** |
| Sodium, mEq/L | 139.5±0.8 | 141.4±0.5 | **1.3%** | 139.9±0.7 | 141.5±0.9 | **1.2%** | 138.5±0.6 | 142.1±1.1 | **2.6%** | **0.07** |
| Potassium, mEq/L | 4.1±0.1 | 4.7±0.1 | **14.8%** | 4.1±0.1 | 4.7±0.2 | **14.0%** | 4.0±0.1 | 4.9±0.1 | **21.7%** | **0.51** |
| Chloride, mEq/L | 102.4±0.5 | 103.0±0.5 | **0.6%** | 103.1±0.8 | 102.8±0.7 | **-0.4%** | 102.1±0.5 | 104.3±0.9 | **2.1%** | **0.06** |
| $CO_2$, mEq/L | 24.9±0.6 | 27.3±0.6 | **9.5%** | 24.8±1.0 | 27.8±0.9 | **12.1%** | 25.3±0.8 | 27.9±0.4 | **10.4%** | **0.85** |
| Phosphorus, mg/dL | 4.0±0.1 | 4.4±0.1 | **10.6%** | 4.2±0.2 | 4.5±0.2 | **6.2%** | 4.1±0.1 | 4.6±0.2 | **11.5%** | **0.44** |
| Protein, g/dL | 6.4±0.2 | 6.5±0.2 | **2.8%** | 6.4±0.1 | 6.7±0.2 | **5.1%** | 6.5±0.2 | 6.6±0.2 | **2.9%** | **0.62** |
| Albumin, g/dL | 4.1±0.1 | 4.2±0.1 | **0.6%** | 4.1±0.1 | 4.3±0.1 | **4.5%** | 4.1±0.1 | 4.2±0.1 | 2.7% | **0.08** |
| Globulin, g/dL | 2.2±0.1 | 2.4±0.1 | **6.7%** | 2.3±0.2 | 2.4±0.2 | **6.0%** | 2.3±0.1 | 2.4±0.1 | 3.2% | **0.93** |
| A:G Ratio[2] | 1.9±0.1 | 1.8±0.1 | **-3.4%** | 1.9±0.2 | 1.8±0.1 | **-2.6%** | 1.8±0.1 | 1.8±0.1 | -1.4% | **0.83** |
| Total Bilirubin, mg/dL | 0.6±0.1 | 0.5±0.2 | **-17.8%** | 0.5±0.1 | 0.5±0.1 | **2.6%** | 0.6±0.1 | 0.5±0.1 | -13.3% | **0.10** |
| Direct Bilirubin, mg/dL | 0.2±0.1 | 0.1±0.04 | **-37.4%** | 0.1±0.03 | 0.1±0.04 | **6.0%** | 0.1±0.03 | 0.1±0.04 | -12.8% | **0.21** |
| Alkaline Phosphatase, IU/L | 65.5±7.3 | 66.8±6.9 | **1.9%** | 64.8±7.3 | 74.1±7.6 | **14.5%** | 66.5±7.3 | 62.8±6.7 | -5.6% | **0.18** |
| Lactate Dehydrogenase, IU/L | 161.8±12.6 | 142.4±10.5 | **-12.0%** | 155.9±13.9 | 136.6±11.6 | **-12.3%** | 163.8±11.9 | 151.8±5.2 | -7.3% | **0.86** |
| Aspartate Aminotransferase, | 22.6±1.4 | 20.0±1.1 | **-11.6%** | 23.3±0.9 | 21.3±0.7 | **-8.6%** | 23.9±3.2 | 24.8±2.3 | 3.7% | **0.23** |
| Alanine Aminotransferase, IU/L | 15.4±1.8 | 14.9±2.2 | **-3.3%** | 16.4±1.6 | 16.4±1.7 | **0.0%** | 15.3±1.5 | 16.6±1.8 | 9.0% | **0.18** |
| Gamma-glutamyl | 14.0±1.9 | 14.3±1.4 | **1.8%** | 13.3±1.2 | 14.0±1.0 | **5.7%** | 13.9±1.2 | 13.9±1.0 | 0.0% | **0.50** |
| Iron Binding Capacity, μg/dL | 371.8±28.6 | 377.5±30.3 | **1.5%** | 378.6±32.1 | 395.1±34.7 | **4.4%** | 371.9±31.4 | 385.0±29.8 | 3.5% | **0.68** |
| UIBC, μg/dL | 272.8±39.5 | 317.6±38.3 | **16.5%** | 310.6±42.4 | 335.1±42.1 | **7.9%** | 264.3±33.3 | 312.0±35.9 | 18.1% | **0.44** |
| Iron, μg/dL | 99.0±20.0 | 59.9±12.2 | **-39.5%** | 68.0±13.4 | 53.8±10.8 | **-21.0%** | 107.6±13.7 | 65.0±12.1 | -39.6% | **0.37** |
| Iron Saturation, % | 28.4±5.8 | 17.5±4.2 | **-38.3%** | 20.3±5.0 | 16.9±4.6 | **-16.7%** | 30.1±5.1 | 20.1±4.2 | -33.2% | **0.38** |
| Total Cholesterol, mg/dL | 160.4±8.0 | 162.9±7.1 | **1.6%** | 164.6±10.5 | 176.9±11.8 | **7.4%** | 161.6±9.4 | 163.3±9.3 | 1.0% | **0.25** |
| Triglycerides, mg/dL | 97.4±12.5 | 106.9±17.6 | **9.8%** | 112.6±18.4 | 121.4±22.7 | **7.8%** | 93.1±10.6 | 107.6±16.6 | 15.6% | **0.52** |
| HDL, mg/dL | 52.3±2.7 | 54.0±3.5 | **3.3%** | 51.4±2.8 | 56.4±2.9 | **9.7%** | 52.3±3.1 | 51.3±2.8 | -1.9% | **0.24** |
| LDL, mg/dL | 88.6±7.2 | 88.6±5.9 | **0.0%** | 90.8±7.0 | 96.1±8.1 | **5.9%** | 91.3±5.9 | 90.4±6.8 | -1.0% | **0.18** |
| Cholesterol Ratio | 3.1±0.2 | 3.1±0.2 | **-0.8%** | 3.2±0.2 | 3.2±0.2 | **-2.3%** | 3.1±0.2 | 3.2±0.2 | 3.6% | **0.53** |

[1]Mean±Standard Error of the Mean.
[2]P value for treatment effect of the difference in starting and ending values indicated by % change for each treatment.

**Table 7. Hematology Values Study 2.[1]**

| | CaHMB | | | HMB-Acid Gel Swallow | | | HMB-Acid Gel Sublingual | | | p Value[2] |
|---|---|---|---|---|---|---|---|---|---|---|
| | Before | After | % Change | Before | After | % Change | Before | After | % Change | |
| WBC, $x10^3/\mu L$ | 7.3±0.3 | 6.5±0.3 | **-10.5%** | 6.4±0.3 | 7.1±0.3 | **9.7%** | 7.1±0.4 | 6.8±0.3 | **-3.5%** | **0.10** |
| RBC, $x10^3/\mu L$ | 4.3±0.2 | 4.3±0.2 | **0.7%** | 4.2±0.1 | 4.4±0.1 | **3.5%** | 4.3±0.1 | 4.3±0.1 | **0.1%** | **0.27** |
| Hemoglobin, g/dL | 13.2±0.7 | 13.3±0.6 | **0.7%** | 13.0±0.4 | 13.4±0.4 | **3.2%** | 13.2±0.5 | 13.2±0.5 | **0.4%** | **0.31** |
| Hematocrit, % | 38.1±1.8 | 38.3±1.7 | **0.7%** | 37.4±1.0 | 38.9±1.1 | **4.2%** | 37.5±1.4 | 38.6±1.3 | **2.9%** | **0.42** |
| MCV, fL | 88.4±1.5 | 88.5±1.4 | **0.1%** | 88.1±1.5 | 88.6±1.5 | **0.6%** | 88.4±1.5 | 89.3±1.4 | **1.0%** | **0.21** |
| MCH, pg | 30.6±0.5 | 30.7±0.6 | **0.2%** | 30.6±0.6 | 30.5±0.6 | **-0.3%** | 30.4±0.5 | 30.5±0.5 | **0.3%** | **0.52** |
| MCHC, g/dL | 34.7±0.2 | 34.7±0.2 | **0.0%** | 34.8±0.2 | 34.5±0.2 | **-0.8%** | 34.5±0.2 | 34.5±0.2 | **0.1%** | **0.14** |
| RDW, % | 13.4±0.2 | 13.4±0.1 | **-0.1%** | 13.3±0.2 | 13.2±0.2 | **-0.5%** | 13.3±0.2 | 13.4±0.2 | **0.7%** | **0.66** |
| Platelets, $x10^3/\mu L$ | 228.0±17.6 | 235.9±10.9 | **3.5%** | 229.0±12.9 | 242.4±12.2 | **5.8%** | 233.8±14.2 | 244.3±17.5 | **4.5%** | **0.92** |
| Neutrophils, % | 44.9±3.8 | 45.6±3.3 | **1.7%** | 45.6±2.7 | 46.0±4.4 | **0.8%** | 45.3±5.2 | 42.4±3.5 | **-6.4%** | **0.90** |
| Lymphocytes, % | 44.8±3.2 | 42.5±3.5 | **-5.0%** | 43.6±2.4 | 43.3±3.9 | **-0.9%** | 43.1±4.2 | 45.6±3.4 | **5.8%** | **0.68** |
| Monocytes, % | 6.9±0.9 | 8.5±1.5 | **23.6%** | 7.5±1.1 | 6.9±0.8 | **-8.3%** | 8.3±1.0 | 8.4±0.9 | **1.5%** | **0.24** |
| Eosinophils, % | 2.9±0.5 | 2.6±0.4 | **-8.7%** | 2.8±0.5 | 3.0±0.5 | **9.1%** | 2.5±0.5 | 3.3±0.5 | **30.0%** | **0.57** |
| Basophils, % | 0.6±0.2 | 0.8±0.2 | **20.0%** | 0.5±0.2 | 0.9±0.3 | **75.0%** | 0.6±0.2 | 0.4±0.2 | **-40.0%** | **0.36** |
| Neutrophils, $x10^3\mu L$ | 3.3±0.3 | 3.0±0.2 | **-9.9%** | 2.9±0.2 | 3.3±0.4 | **10.6%** | 3.3±0.5 | 2.9±0.3 | **-12.1%** | **0.49** |
| Lymphocytes, $x10^3/\mu L$ | 3.3±0.3 | 2.8±0.3 | **-13.5%** | 2.8±0.2 | 3.1±0.3 | **9.4%** | 3.0±0.3 | 3.1±0.3 | **5.0%** | **0.06** |
| Monocytes, $x10^3/\mu L$ | 0.5±0.1 | 0.5±0.1 | **5.0%** | 0.5±0.1 | 0.5±0.04 | **2.6%** | 0.6±0.05 | 0.6±0.05 | **0.0%** | **0.71** |
| Eosinophils, $x\ 10^3/_{ll}L$ | 0.2±0.04 | 0.2±0.03 | **-12.5%** | 0.2±0.03 | 0.2±0.04 | **13.3%** | 0.2±0.04 | 0.2±0.03 | **35.7%** | **0.42** |
| Basophils, $x10^3/\mu L$ | 0.06±0.02 | 0.06±0.02 | **0.0%** | 0.05±0.02 | 0.09±0.03 | **75.0%** | 0.06±0.02 | 0.04±0.0 | **-40.0%** | **0.29** |

[1]Mean±Standard Error of the Mean.

[2]P value for treatment effect of the difference in starting and ending values indicated by % change for each treatment.

Example 2

In this example the effect of administration of HMB-acid gel is compared to that of calcium HMB on muscle damage after an eccentric bout of exercise. As shown in Example 1, peak plasma HMB levels and HMB clearance rate are increased with HMB free acid gel administration compared with CaHMB (13). This example shows that the quicker response of HMB administered as free acid gel prior to and following a bout of extreme exercise protects the muscle from damage better than HMB administered as the calcium HMB salt.

***Effects of HMB and exercise on markers of muscle damage and inflammatory factors:*** Strenuous exercise, such as resistance training or maximal effort exercise, causes an increase in leakage of the enzyme creatine phosphokinase (CPK) from muscle cells (21; 31). Human studies have shown that muscle damage following intense exercise, measured by elevated plasma CPK is reduced with calcium HMB supplementation (14; 22; 26; 33). A study on muscle damage after a prolonged 20 km run on a collegiate cross country course with both inclines and declines also showed chronic calcium HMB administration is effective in decreasing the rise in plasma CPK over a 4 day period following the run (23). Nissen et al. (26) demonstrated a dosage effect of calcium HMB (with 3.0 gram per day being more effective than 1.5 grams per day) in decreasing CPK as well as resulting in significant reductions in urinary 3-methylhistidine (3-MH, a well established indicator of myofibrillar protein degradation (39)). Gallagher et al. supplemented dosages of HMB to 37 male college students, performing resistance exercise training, corresponding to 3.0 (38 mg/kg body weight-$d^{-1}$) and 6.0 g (76 mg/kg body weight-$d^{-1}$) of calcium HMB per day. Both doses had similar effects on improving lean mass and strength gains, however, the higher dosage resulted in significant improvements in minimizing CPK leakage suggesting significant amelioration in muscle damage following exercise. These observations indicate that higher plasma levels of HMB appear to more protective of muscle damage following exercise.

As shown in Example 1, HMB as a free acid (in a gel) is absorbed faster than calcium HMB, results in higher plasma levels of HMB, and is cleared more readily by muscle.

Methods:

***Subjects:*** We conducted a study at the Iowa State University Health and Human Performance Laboratory; this study was approved by the Iowa State University Institutional Review Board, and was registered at ClinicalTrials.gov (NCT01150526). We recruited 12 men and 13 women between the ages of 20 and 36 from the Iowa State University community and surrounding area.

***Treatments****:* The experimental design is depicted in Figure 8.

Five treatments were administered as follows:

Treatment 1: Placebo. This group received a placebo capsule and a placebo syringe dosage at each dosage administration.

Treatment 2: CaHMB pre-exercise. This group received a CaHMB capsule and a placebo syringe dosage 30 min prior to the acute exercise bout. The remaining additional dosages during the study consisted of one placebo capsule and one placebo syringe dosage.

Treatment 3: HMB-acid gel pre-exercise. This group received a placebo capsule and a syringe dosage of HMB-acid gel 30 min prior to the acute exercise bout. The remaining additional dosages during the study consisted of one placebo capsule and one placebo syringe dosage.

Treatment 4: CaHMB pre- and post-exercise. This group received a CaHMB capsule and a placebo syringe dosage at all administration times during the study.

Treatment 5: HMB-acid gel pre- and post-exercise. This group received a syringe dosage of HMB- acid gel and one placebo capsule at all administration times during the study.

To blind the treatments to both the researchers and subjects, each subject took a capsule and syringe dosage of supplement at each administration. The capsules contained either one gram of calcium lactate (Placebo) or one gram of calcium β-hydroxy-β-methylbutryate (CaHMB). The syringe dosages were formulated to be similar in taste and appearance and contained either 0.8 g of corn syrup (Placebo) or 0.8 g of β-hydroxy-β-methylbutryate free acid, the same amount of HMB as in the capsule dosage. The 3 daily doses provided the same total HMB dosage (2.4 g as calcium HMB in the capsules or 2.4 g as HMB free acid in the syringes).

***Eccentric Exercise Bout****:* Subjects refrained from vigorous exercise for three days before reporting to the laboratory. All subjects were studied after an overnight fast. Subjects had a fasting blood sample taken and a spot urine

sample was collected. Subjects then consumed their assigned supplement and 30 minutes later the eccentric exercise session was performed. The exercise consisted of 50 maximal effort contractions of knee extensors while attempting to resist the Biodex lever arm as it moves the knee joint from full extension to 90 degrees flexion; this is similar to letting a heavy weight down slowly while in a seated position. Each contraction lasted about 2 seconds and 12 seconds were allowed between contractions. This protocol was performed on the right leg followed by the left leg. The subjects received 2 more daily dosages of treatment (placebo or HMB) with instructions to take the dosages at lunch and dinner. For the next 4 days the subjects returned to the laboratory fasted each morning, had blood taken and urine collected and then consumed the morning dosage of their supplement. The subjects were again given the 2 remaining daily supplement dosages with instructions to take them at lunch and dinner.

***Serum and Urine Samples:*** A fasting blood sample was taken from a superficial forearm vein each morning when the subjects reported to the laboratory for testing. Additionally a clean catch urine sample was collected at this time. Serum CPK was analyzed by a commercial laboratory (Quest Diagnostics, Madison NJ). Urine 3-Methylhistidine (3MH) was analyzed by a previously published GC/MS method (9). Urinary creatinine was analyzed by colorimetric assay (Cayman Chemical Company, Ann Arbor, MI). The urinary 3MH data was normalized to urinary creatinine and expressed as 3MH:creatinine ratio, μmol:mg.

***Statistics:*** Data were analyzed using the mixed model procedure in Statistical Analysis System for Windows (Release 9.1.3, SAS Institute, Cary, NC). The change in each variable was analyzed at each measurement time point. The model included the baseline or time 0 value as a covariate and included gender and treatment as main effects. CPK data were transformed using the Rank procedure in SAS before analysis. Contrasts were used to compare the HMB-acid gel pre- and post exercise mean to the other treatment means.

**Results:**

The subjects' demographics are shown in Table 8. The age, height, and weight of the subjects by treatment were similar.

**Table 8. Subject Characteristics**

| | Treatment 1 | Treatment 2 | Treatment 3 | Treatment 4 | Treatment 5 |
|---|---|---|---|---|---|
| N | 5 | 4 | 5 | 5 | 6 |
| M/F | 3/2 | 2/2 | 2/3 | 2/3 | 3/3 |
| Age (y) | 25.2±2.5 | 21.0±0.4 | 24.0±1.4 | 25.8±2.6 | 22.2±1.0 |
| Height (cm) | 173±2.7 | 173±5.5 | 170±6.0 | 173±6.0 | 171±3.1 |
| Weight (kg) | 67.0±2.3 | 68.9±9.8 | 68.1±5.8 | 69.7±6.9 | 65.2±5.1 |

[a]Data are presented as Mean±SEM.

Treatments were: (Treatment 1): placebo capsule and placebo syringe dosage; (Treatment 2) Calcium HMB capsule and placebo syringe dosage pre-exercise followed by placebo capsule and placebo syringe dosage post exercise; (Treatment 3): Placebo capsule and HMB- acid gel syringe dosage given pre-exercise followed by placebo capsule and placebo syringe dosage post exercise; (Treatment 4): Calcium HMB capsule and placebo syringe dosage pre- and post exercise; and (Treatment 5): Placebo capsule and HMB-acid gel syringe dosage given pre- and post exercise. Treatments were administered 3 times daily, 30 min before the morning testing and then again at approximately noon and 6 PM.

Serum CPK and urinary 3MH:urinary creatinine ratios are shown in Table 9. There were no differences in baseline (time 0) values. The eccentric exercise caused up to a four-fold increase in serum CPK, indicating muscle membrane damage. However, HMB-acid gel administered both pre-and post exercise (Treatment 5), blunted this increase by up to 64% ($P < 0.03$) at 24 h post exercise and 86% 48 h post exercise ($P < 0.005$). Continuing at 72 h the increase in CPK still tended to be less with the HMB-acid gel treatment. Figure 9 illustrates the rise and fall in CPK values over the course of the study. Also shown in Table 2 is the effect of the treatments on 3MH:Cr ratio, a measure of protein degradation in muscle. Although these data only are indicative of a trend, the data do show no increase and even a decrease in protein degradation in the HMB-acid gel pre- and post exercise treatment. This would indicate a decrease in protein degradation resulting in more favorable protein turnover and protein accretion in muscle.

**Table 9. Serum Creatine Phosphokinase (CPK) and Urinary 3-Methylhistidine to Urinary Creatinine Ratio (3MH:Cr) After an Acute Bout of Exercise**

| | Treatment 1 | Treatment 2 | Treatment 3 | Treatment 4 | Treatment 5 | P-value[b] |
|---|---|---|---|---|---|---|
| CPK, U/mL | | | | | | |

(continued)

| | Treatment 1 | Treatment 2 | Treatment 3 | Treatment 4 | Treatment 5 | P-value[b] |
|---|---|---|---|---|---|---|
| Baseline | 132±35 | 95±12 | 107±17 | 127±38 | 146±43 | |
| at 24 h | 356±108 | 375±68 | 434±126 | 416±157 | 264±75 | 0.03 |
| at 48 h | 253±73 | 246±38 | 275±69 | 228±73 | 170±41 | 0.005 |
| at 72 h | 197±48 | 231±59 | 209±54 | 223±52 | 180±34 | 0.09 |
| at 96 h | 169±36 | 281±105 | 181±33 | 214±52 | 187±46 | 0.51 |
| 3MH:Cr, μmol/mg | | | | | | |
| Baseline | 0.233±0.066 | 0.164±0.037 | 0.173±0.028 | 0.205±0.025 | 0.225±0.034 | |
| at 24 h | 0.211±0.035 | 0.190±0.027 | 0.213±0.037 | 0.203±0.039 | 0.213±0.025 | 0.88 |
| at 48 h | 0.238±0.033 | 0.240±0.052 | 0.190±0.014 | 0.220±0.027 | 0.208±0.012 | 0.62 |
| at 72 h | 0.251±0.046 | 0.204±0.058 | 0.172±0.013 | 0.231±0.037 | 0.175±0.013 | 0.14 |
| at 96 h | 0.231±0.044 | 0.187±0.029 | 0.202±0.020 | 0.269±0.038 | 0.186±0.043 | 0.27 |

[a]Data are presented as Mean±SEM.
Treatments were: (Treatment 1): placebo capsule and placebo syringe dosage; (Treatment 2) Calcium HMB capsule and placebo syringe dosage pre-exercise followed by placebo capsule and placebo syringe dosage post exercise; (Treatment 3): Placebo capsule and HMB free acid gel syringe dosage given pre-exercise followed by placebo capsule and placebo syringe dosage post exercise; (Treatment 4): Calcium HMB capsule and placebo syringe dosage pre- and post exercise; and (Treatment 5): Placebo capsule and HMB free acid gel syringe dosage given pre- and post exercise. Treatments were administered 3 times daily, 30 min before the morning testing and then again at approximately noon and 6 PM.
[b]P-value HMB Free acid gel (Treatment 5) contrasted with the other treatments.

**Implications:**

Based upon the observations in Example 1, muscle tissue was exposed to much higher levels of serum HMB when HMB free acid was administered. Additionally, in Example 1 it was shown that clearance of HMB from serum to muscle and tissues was also much greater when HMB-acid was administered compared with CaHMB administration. Thus, in Example 2 it is shown that this additional utilization of HMB by muscle in the free acid form is more protective of the muscle tissue after an acute bout of exercise than HMB administered in the calcium form.

The foregoing description and drawings comprise illustrative embodiments of the present invention. The foregoing embodiments and the methods described herein may vary based on the ability, experience, and preference of those skilled in the art. Merely listing the steps of the method in a certain order does not constitute any limitation on the order of the steps of the method. Those skilled in the art who have the disclosure before them will be able to make modifications and variations therein without departing from the scope of the invention. Administration of the composition of the present invention will be in an amount sufficient to achieve a desired effect as recognized by one of ordinary skill in the art.

Reference List

1. The Body Fluid Compartments: Extracellular and Intracellular Fluids; Interstitial Fluid and Edema. In: Textbook of Medical Physiology, edited by Guyton AC and Hall JE. Philadelphia: W B Saunders Company, 2007.

2. Adamson LF and Greenberg DM. The significance of certain carboxylic acids as intermediates in the biosynthesis of cholesterol. Biochim Biophys Acta 23: 472-479, 1957.

3. Bachhawat BK, Robinson WG and Coon MJ. The enzymatic cleavage of beta-hydroxy-beta-methylglutaryl coenzyme a to aceto-acetate and acetyl coenzyme A. J Biol Chem 216: 727-736, 1955.

4. Bloch K, Clark LC and Haray I. Utilization of branched chain acids in cholesterol synthesis. J Biol Chem 211: 687-699, 1954.

5. Clark RH, Feleke G, Din M, Yasmin T, Singh G, Khan F and Rathmacher JA. Nutritional treatment for acquired

immunodeficiency virus-associated wasting using β-hydroxy-β-methylbutyrate, glutamine and arginine: A randomized, double-blind, placebo-controlled study. JPEN J Parenter Enteral Nutr 24(3): 133-139, 2000.

6. Coon MJ. Enzymatic synthesis of branched chain acids from amino acids. Fed Proc 14: 762-764, 1955.

7. Dreyer HC, Drummond MJ, Pennings B, Fujita S, Glynn EL, Chinkes DL, Dhanani S, Volpi E and Rasmussen BB. Leucine-enriched essential amino acid and carbohydrate ingestion following resistance exercise enhances mTOR signaling and protein synthesis in human muscle. Am J Plysiol Endocrinol Metab 294: E392-E400, 2008.

8. Dreyer HC, Fujita S, Cadenas JG, Chinkes DL, Volpi E and Rasmussen BB. Resistance exercise increases AMPK activity and reduces 4E-BP1 phosphorylation and protein synthesis in human skeletal muscle. J Plysiol 576: 613-624, 2006.

9. Eley HL, Russell ST, Baxter JH, Mukherji P and Tisdale MJ. Signaling pathways initiated by β-hydroxy-β-methylbutyrate to attenuate the depression of protein synthesis in skeletal muscle in response to cachectic stimuli. Am J Plysiol Endocrinol Metab 293: E923-E931, 2007.

10. Eley HL, Russell ST and Tisdale MJ. Attenuation of depression of muscle protein synthesis induced by lipopolysaccharide, tumor necrosis factor and angiotensin II by β-hydroxy-β-methylbutyrate. Am J Physiol Endocrinol Metab 295: E1409-E1416, 2008.

11. Eley HL, Russell ST and Tisdale MJ. Mechanism of Attenuation of Muscle Protein Degradation Induced by Tumor Necrosis Factor Alpha and Angiotensin II by beta-Hydroxy-beta-methylbutyrate. Am J Physiol Endocrinol Metab 295: E1417-E1426,2008.

12. Eubanks May P, Barber A, Hourihane A, D'Olimpio JT and Abumrad NN. Reversal of cancer-related wasting using oral supplementation with a combination of β-hydroxy-β-methylbutyrate, arginine, and glutamine. Am J Surg 183: 471-479,2002.

13. Fuller, J. C., Jr., Sharp, R. L., Angus, H. F., Baier, S. M., and Rathmacher, J. A. Free acid gel form of β-hydroxy-β-methylbutyrate (HMB) improves HMB clearance from plasma in humans compared to the calcium HMB salt. Br.J.Nutr. 2010. Ref Type: In Press

14. Gallagher PM, Carrithers JA, Godard MP, Schulze KE and Trappe SW. β-Hydroxy-β-methylbutyrate ingestion, Part I: Effects on strength and fat free mass. Med Sci Sports Exerc 32(12): 2109-2115, 2000.

15. Gallagher PM, Carrithers JA, Godard MP, Schutze KE and Trappe SW. β-Hydroxy-β-methylbutyrate ingestion, Part II: Effects on hematology, hepatic, and renal function. Med Sci Sports Exerc 32(12): 2116-2119, 2000.

16. Gey KF, Pletsher A, Isler O, Ruegg R and Wursch J. Influence of iosoprenoid C5 and C6 compounds on the incorporation of acetate in cholesterol. Helvetica Chim Acta 40: 2354-2368, 1957.

17. Gey, K. F., Pletsher, A., Isler, O., Ruegg, R., and Wursch, J. The influence of isoperenic C5 and C6 compounds upon the acetate incorporation into cholesterol. Helvetica Chim.Acta 40, 2369. 1957. b
Ref Type: Abstract

18. Harper AE, Benevenga NJ and Wohlhueter RM. Effects of ingestion of disproportionate amounts of amino acids. Physiol Rev 53: 428-558, 1970.

19. Hill, D. S., Szewczyk, N., Brookfield, R., Loughna, P., Rathmacher, J., Rennie, M. J., and Atherton, P. J. β-hydroxy-β-methylbutyrate (HMB) stimulates mammalian target of rapamycin complex 1 (mTORc1) signaling via a phosphatidylinositol-3-kinase (PI3K)-dependent pathway. FASEB J. 2011.
Ref Type: Abstract

20. Isler, O., Ruegg, R., Wursch, J., Gey, K. F., and Pletsher, A. Biosynthesis of cholesterol from β,τ-dihydroxy-β-methylvaleric acid. Helvetica Chim.Acta 40, 2369. 1957.
Ref Type: Abstract

21. Janssen GME, Kuipers H, Willems GM, Does RJMM, Janssen MPE and Geurten P. Plasma activity of muscle enzymes: quantification of skeletal muscle damage and relationship with metabolic variables. Int J Sport 10: S160-S168, 1989.

22. Jówko E, Ostaszewski P, Jank M, Sacharuk J, Zieniewicz A, Wilczak J and Nissen S. Creatine and β-hydroxy-β-methylbutyrate (HMB) additively increases lean body mass and muscle strength during a weight training program. Nutr 17: 558-566, 2001.

23. Knitter AE, Panton L, Rathmacher JA, Petersen A and Sharp R. Effects of β-hydroxy-β-methylbutyrate on muscle damage following a prolonged run. J Appl Physiol 89(4): 1340-1344, 2000.

24. Krebs HA and Lund P. Aspects of the regulation of the metabolism of branched-chain amino acids. Advan Enzyme Regul 15: 375-394, 1977.

25. Nissen S, Panton L, Sharp RL, Vukovich M, Trappe SW and Fuller JC, Jr. β-Hydroxy-β-methylbutyrate (HMB) supplementation in humans is safe and may decrease cardiovascular risk factors. J Nutr 130: 1937-1945, 2000.

26. Nissen S, Sharp R, Ray M, Rathmacher JA, Rice J, Fuller JC, Jr., Connelly AS and Abumrad NN. Effect of the leucine metabolite β-hydroxy β-methylbutyrate on muscle metabolism during resistance-exercise training. JAppl Plysiol 81(5): 2095-2104, 1996.

27. Nissen S, Van Koevering M and Webb D. Analysis of β-Hydroxy-β-methyl Butyrate in Plasma by Gas Chromatography and Mass Spectrometry. Anal Biochem 188(1): 17-19, 1990.

28. Nissen S, Van Koevering M and Webb D. Analysis of β-hydroxy-β-methyl butyrate in plasma by gas chromatography and mass spectrometry. Anal Biochem 188: 17-19, 1990.

29. Nissen SL and Abumrad NN. Nutritional role of the leucine metabolite β-hydroxy-β-methylbutyrate (HMB). J Nutr Biochem 8: 300-311, 1997.

30. Nissen SL and Sharp RL. Effect of dietary supplements on lean mass and strength gains with resistance exercise: a meta-analysis. J Appl Plysiol 94: 651-659, 2003.

31. Nuviala RJ, Roda L, Lapieza MG, Boned B and Giner A. Serum enzymes activities at rest and after a marathon race. J Sports Med Phys Fitness 32: 180-186, 1992.

32. Ostaszewski P, Kostiuk S, Balasinska B, Jank M, Papet I and Glomot F. The leucine metabolite 3-hydroxy-3-methylbutyrate (HMB) modifies protein turnover in muscles of the laboratory rats and domestic chicken in vitro. J Anim Plysiol Anim Nutr (Swiss) 84: 1-8, 2000.

33. Panton LB, Rathmacher JA, Baier S and Nissen S. Nutritional supplementation of the leucine metabolite β-hydroxy β-methylbutyrate (HMB) during resistance training. Nutr 16(9): 734-739, 2000.

34. Phillips SM, Tipton KD, Aarsland A, Wolf SE and Wolfe RR. Mixed muscle protein synthesis and breakdown after resistance exercise in humans. Am J Physiol 273: E99-107, 1997.

35. Plaut GWE and Lardy HA. Enzymatic incorporation of C14-bicarbonate into acetoacetate in the presence of various substrates. J Biol Chem 192: 435-445, 1951.

36. Rabinowitz JL, Dituri F, Cobey F and Gurin S. Branched chain acids in the biosynthesis of squalene and cholesterol. Fed Proc 14: 760-761, 1955.

37. Rathmacher JA, Link GA, Flakoll PJ and Nissen SL. Gas chromatographic-mass spectrometric analysis of stable isotopes of 3-methylhistidine in biological fluids: application to plasma kinetics in vivo. Biol Mass Spectrom 21: 560-566, 1992.

38. Rathmacher JA, Nissen S, Panton L, Clark RH, Eubanks MP, Barber AE, D'Olimpio J and Abumrad NN. Supplementation with a combination of beta-hydroxy-beta-methylbutyrate (HMB), arginine, and glutamine is safe and

could improve hematological parameters. JPEN J Parenter Enteral Nutr 28: 65-75, 2004.

39. Rathmacher JA and Nissen SL. Development and application of a compartmental model of 3-methylhistidine metabolism in humans and domestic animals. Adv Exp Med Biol 445: 303-324, 1998.

40. Rennie MJ. Exercise- and nutrient-controlled mechanisms involved in maintenance of the musculoskeletal mass. Biochem Soc Trans 35: 1302-1305, 2007.

41. Robinson WG, Bachhawat BK and Coon MJ. Enzymatic carbon dioxide fixation by senecioyl coenzyme A. Fed Proc 13: 281, 1954.

42. Rudney H. The synthesis of β-hydroxy-β-methylglutaric acid in rat liver homogenates. J Am Chem Soc 76: 2595, 1954.

43. Rudney H and Farkas TG. Biosynthesis of branched chain acids. Fed Proc September: 757-759, 1955.

44. Russell ST and Tisdale MJ. Mechanism of attenuation by beta-hydroxy-beta-methylbutyrate of muscle protein degradation induced by lipopolysaccharide. Mol Cell Biochem 330(1-2): 171-179, 2009.

45. **SAS Institute Inc.** *SAS User's Guide: Statistics.* Cary,NC: SAS Institute Inc., 1985.

46. Slater G, Jenkins D, Logan P, Lee H, Vukovich MD, Rathmacher JA and Hahn AG. b-hydroxy b-methylbutyrate (HMB) supplementation does not affect changes in strength or body composition during resistance training in trained men. Int J Sport Nutr Exerc Metab 11: 384-396, 2001.

47. Smith HJ, Mukerji P and Tisdale Mj. Attenuation of proteasome-induced proteolysis in skeletal muscle by β-hydroxy-β-methylbutyrate in cancer-induced muscle loss. Cancer Res 65: 277-283, 2005.

48. Smith HJ, Wyke SM and Tisdale MJ. Mechanism of the attenuation of proteolysis-inducing factor stimulated protein degradation in muscle by beta-hydroxy-beta-methylbutyrate. Cancer Res 64: 8731-8735, 2004.

49. Sousa MF, Abumrad NN, Martins C, Nissen S and Riella MC. Calcium β-hydroxy-β-methylbutyrate. Potential role as a phosphate binder in uremia: In vitro study. Nephron 72: 391-394, 1996.

50. Thalhammer F, Schenk P, Burgmann H, El M, I, Hollenstein UM, Rosenkranz AR, Sunder-Plassmann G, Breyer S and Ratheiser K. Single-dose pharmacokinetics of meropenem during continuous venovenous hemofiltration. Antimicrob Agents Chemother 42: 2417-2420, 1998.

51. Urso R, Blardi P and Giorgi G. A short introduction to pharmacokinetics. Eur Rev Med Pharmacol Sci 6: 33-44, 2002.

52. Vukovich MD, Slater G, Macchi MB, turner MJ, Fallon K, Boston T and Rathmacher J. β-Hydroxy-β-methylbutyrate (HMB) kinetics and the influence of glucose ingestion in humans. J Nutr Biochem 12: 631-639, 2001.

53. Vukovich MD, Stubbs NB and Bohlken RM. Body composition in 70-year old adults responds to dietary β-hydroxy-β-methylbutyrate (HMB) similar to that of young adults. J Nutr 131(7): 2049-2052, 2001.

54. Zabin I and Bloch K. The utilization of butyric acid for the synthesis of cholesterol and fatty acids. J Biol Chem 192: 261-266, 1951.

## Claims

**1.** A composition comprising beta-hydroxy-beta-methylbutyric acid (HMB) in free acid form (HMB-acid) for use in improving the effectiveness of HMB upon administration,compared to the administration of a similar dosage of a calcium salt HMB composition, in decreasing muscle damage in muscle subjected to stress, decreasing muscle damage in muscle subjected to damage, improving a person's immune response or decreasing disease-associated wasting.

2. A composition for use according to claim 1 wherein the HMB-acid is formulated as a gel or a liquid.

3. A composition according to claim 1 or claim 2 in the form of an oral composition.

4. A composition according to any one of claims 1 to 3 in the form of a sublingual composition.

5. A composition for use according to any preceding claim wherein the HMB-acid is present in an amount of from 0.5 grams to 30 grams.

6. A composition for use according to claim 5 wherein the HMB-acid is administered one time per twenty-four hour period.

7. A composition according to any one of claims 1 to 6 wherein the HMB-acid is administered in an amount of from 0.01 to 0.2 grams of HMB-acid per kilogram body weight per twenty-four hours.

8. A non-therapeutic method for improving nitrogen retention, improving protein sparing, improving lean body mass, improving muscle function, improving muscle performance, improving a person's lipid profile or improving a person's emotional state comprising administering beta-hydroxy-beta-methylbutyric acid (HMB) in free acid form (HMB-acid)

9. A method according to claim 8 wherein the HMB-acid is formulated as a gel or a liquid.

10. A method according to claim 8 or claim 9 wherein the HMB-acid is in the form of an oral composition or sublingual composition.

11. A method according to any one of claims 8 to 10 wherein the HMB-acid is present in an amount of from 0.5 grams to 30 grams.

12. A method according to claim 11 wherein the HMB-acid is administered one time per twenty-four hour period.

13. A method according to any one of claims 8 to 12 wherein the HMB-acid is administered in an amount of from 0.01 to 0.2 grams of HMB-acid per kilogram body weight per twenty-four hours.

14. A composition comprising beta-hydroxy-beta-methylbutyric acid (HMB) in free acid form (HMB-acid) as a gel formulation.

15. A composition comprising beta-hydroxy-beta-methylbutyric acid (HMB) in free acid form (HMB-acid) as a liquid formulation

16. A composition according to claim 14 or 15 in the form of an oral composition or a sublingual composition.

## Patentansprüche

1. Zusammensetzung, umfassend Beta-hydroxy-beta-methylbuttersäure (HMB) in freier Säureform (HMB-Säure) zur Verwendung bei der Verbesserung der Wirksamkeit von HMB bei Verabreichung, verglichen mit der Verabreichung einer ähnlichen Dosis einer Calciumsalz-HMB-Zusammensetzung, zur Verringerung von Muskelschädigung von Stress ausgesetztem Muskel, Verringerung von Muskelschädigung von Schädigung ausgesetztem Muskel, Verbesserung der Immunantwort einer Person oder Verringerung von mit Krankheit verbundener Auszehrung.

2. Zusammensetzung zur Verwendung nach Anspruch 1, wobei die HMB-Säure als ein Gel oder eine Flüssigkeit formuliert ist.

3. Zusammensetzung nach Anspruch 1 oder Anspruch 2 in Form einer oralen Zusammensetzung.

4. Zusammensetzung nach einem der Ansprüche 1 bis 3 in Form einer sublingualen Zusammensetzung.

5. Zusammensetzung zur Verwendung nach einem der vorhergehenden Ansprüche, wobei die HMB-Säure in einer Menge von zwischen 0,5 Gramm bis 30 Gramm vorhanden ist.

6. Zusammensetzung zur Verwendung nach Anspruch 5, wobei die HMB-Säure während eines Zeitraums von vierundzwanzig Stunden einmal verabreicht wird.

7. Zusammensetzung nach einem der Ansprüche 1 bis 6, wobei die HMB-Säure in einer Menge von 0,01 bis 0,2 Gramm von HMB-Säure pro Kilogramm Körpergewicht pro vierundzwanzig Stunden verabreicht wird.

8. Nichttherapeutisches Verfahren zur Verbesserung von Stickstoffzurückhaltung, Verbesserung von Proteineinsparung, Verbesserung von fettfreier Körpermasse, Verbesserung von Muskelfunktion, Verbesserung von Muskelleistung, Verbesserung des Lipidprofils einer Person oder Verbesserung des emotionalen Zustands einer Person, das das Verabreichen von Beta-hydroxy-beta-methylbuttersäure (HMB) in freier Säureform (HMB-Säure) umfasst.

9. Verfahren nach Anspruch 8, wobei die HMB-Säure als ein Gel oder eine Flüssigkeit formuliert ist.

10. Verfahren nach Anspruch 8 oder Anspruch 9, wobei die HMB-Säure in Form einer oralen Zusammensetzung oder sublingualen Zusammensetzung ist.

11. Verfahren nach einem der Ansprüche 8 bis 10, wobei die HMB-Säure in einer Menge von zwischen 0,5 Gramm bis 30 Gramm vorhanden ist.

12. Verfahren nach Anspruch 11, wobei die HMB-Säure während eines Zeitraums von vierundzwanzig Stunden einmal verabreicht wird.

13. Verfahren nach einem der Ansprüche 8 bis 12, wobei die HMB-Säure in einer Menge von 0,01 bis 0,2 Gramm von HMB-Säure pro Kilogramm Körpergewicht pro vierundzwanzig Stunden verabreicht wird.

14. Zusammensetzung, die Beta-hydroxy-beta-methylbuttersäure (HMB) in freier Säureform (HMB-Säure) als Gelformulierung umfasst.

15. Zusammensetzung, die Beta-hydroxy-beta-methylbuttersäure (HMB) in freier Säureform (HMB-Säure) als Flüssigformulierung umfasst.

16. Zusammensetzung nach Anspruch 14 oder 15 in Form einer oralen Zusammensetzung oder einer sublingualen Zusammensetzung.

## Revendications

1. Composition comprenant de l’acide bêta-hydroxy-bêta-méthylbutyrique (HMB) sous forme d’acide libre (acide HMB) destinée à être utilisée pour améliorer l’efficacité de HMB lors de son administration, par rapport à l’administration d’un dosage similaire d’une composition de sel de calcium de HMB, en diminuant les lésions musculaires dans un muscle soumis à une tension, en diminuant les lésions musculaires dans un muscle sujet à des lésions, en améliorant une réponse immunitaire d’une personne ou en diminuant l’émaciation associée à une maladie.

2. Composition destinée à être utilisée selon la revendication 1, ledit acide HMB étant formulé sous forme de gel ou de liquide.

3. Composition selon la revendication 1 ou la revendication 2 sous la forme d’une composition orale.

4. Composition selon l’une quelconque des revendications 1 à 3 sous la forme d’une composition sublinguale.

5. Composition destinée à être utilisée selon l’une quelconque des revendications précédentes, ledit acide HMB étant présent en une quantité allant de 0,5 gramme à 30 grammes.

6. Composition destinée à être utilisée selon la revendication 5, ledit acide HMB étant administré une seule fois par période de vingt-quatre heures.

7. Composition selon l’une quelconque des revendications 1 à 6, ledit acide HMB étant administré en une quantité allant de 0,01 à 0,2 gramme d’acide HMB par kilogramme de poids corporel par vingt-quatre heures.

**8.** Méthode non-thérapeutique permettant d'augmenter la rétention d'azote, d'augmenter l'épargne protéique, d'augmenter la masse corporelle maigre, d'améliorer la fonction musculaire, d'améliorer les performances musculaires, d'améliorer le profil lipidique d'une personne ou d'améliorer l'état émotionnel d'une personne comprenant l'administration d'acide bêta-hydroxy-bêta-méthylbutyrique (HMB) sous forme d'acide libre (acide HMB).

**9.** Méthode selon la revendication 8, ledit acide HMB étant formulé sous forme de gel ou de liquide.

**10.** Méthode selon la revendication 8 ou la revendication 9, ledit acide HMB étant sous la forme d'une composition orale ou d'une composition sublinguale.

**11.** Méthode selon l'une quelconque des revendications 8 à 10, ledit acide HMB étant présent en une quantité allant de 0,5 gramme à 30 grammes.

**12.** Méthode selon la revendication 11, ledit acide HMB étant administré une fois par période de vingt-quatre heures.

**13.** Méthode selon l'une quelconque des revendications 8 à 12, ledit acide HMB étant administré en une quantité allant de 0,01 à 0,2 gramme d'acide HMB par kilogramme de poids corporel par vingt-quatre heures.

**14.** Composition comprenant de l'acide bêta-hydroxy-bêta-méthylbutyrique (HMB) sous forme d'acide libre (acide HMB) sous la forme d'une formulation en gel.

**15.** Composition comprenant de l'acide bêta-hydroxy-bêta-méthylbutyrique (HMB) sous forme d'acide libre (acide HMB) sous la forme d'une formulation liquide.

**16.** Composition selon la revendication 14 ou 15 sous la forme d'une composition orale ou d'une composition sublinguale.

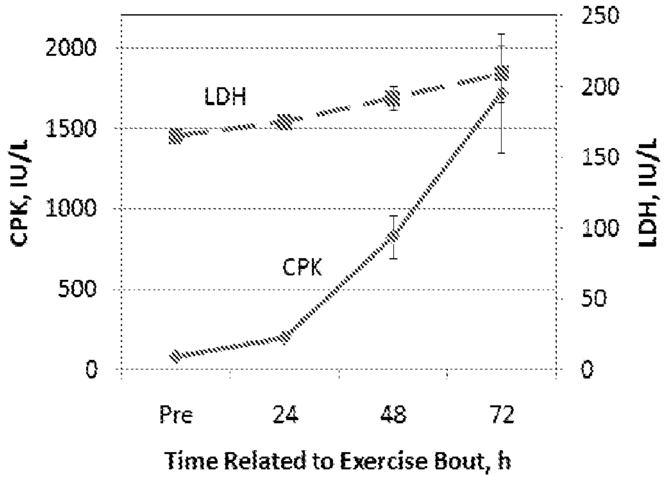
CPK, IU/L
LDH, IU/L
LDH
CPK
2000
1500
1000
500
0
250
200
150
100
50
0
Pre
24
48
72
Time Related to Exercise Bout, h

FIG. 1

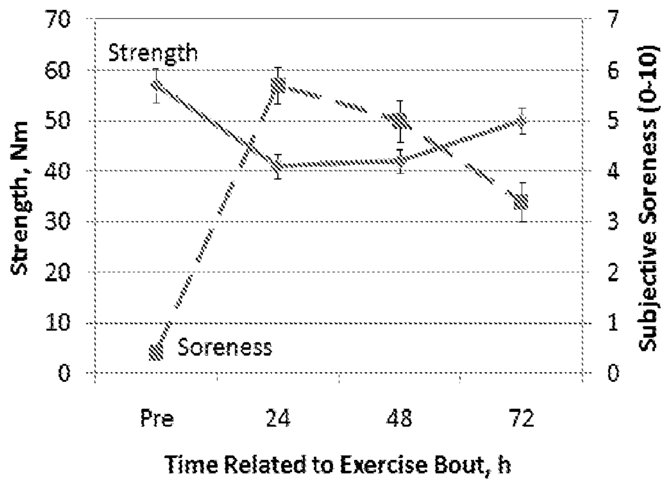
Strength, Nm
Subjective Soreness (0-10)
Strength
Soreness
70
60
50
40
30
20
10
0
7
6
5
4
3
2
1
0
Pre
24
48
72
Time Related to Exercise Bout, h

FIG. 2

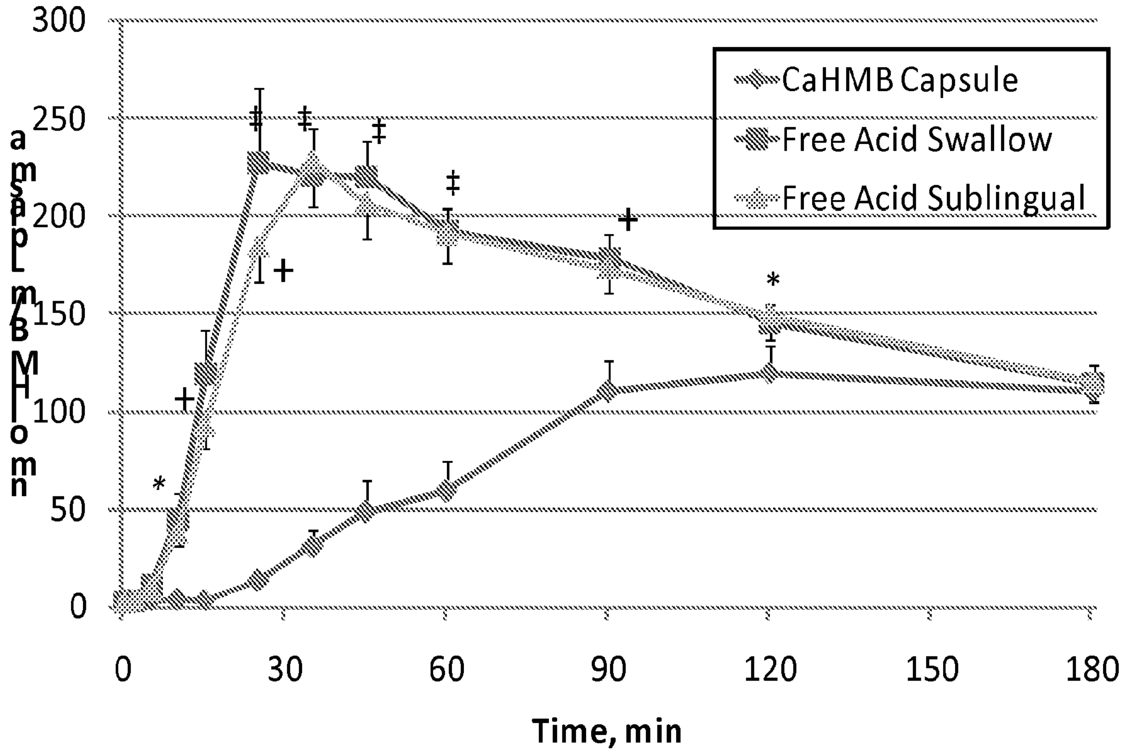
300
250
200
150
100
50
0
0
30
60
90
120
150
180
Time, min
CaHMB Capsule
Free Acid Swallow
Free Acid Sublingual

FIG. 3

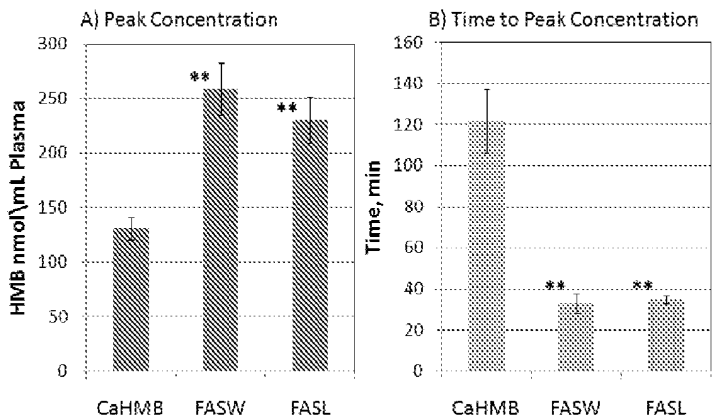
A) Peak Concentration
HMB nmol\mL Plasma
CaHMB
FASW
FASL
B) Time to Peak Concentration
Time, min
CaHMB
FASW
FASL

FIG. 4

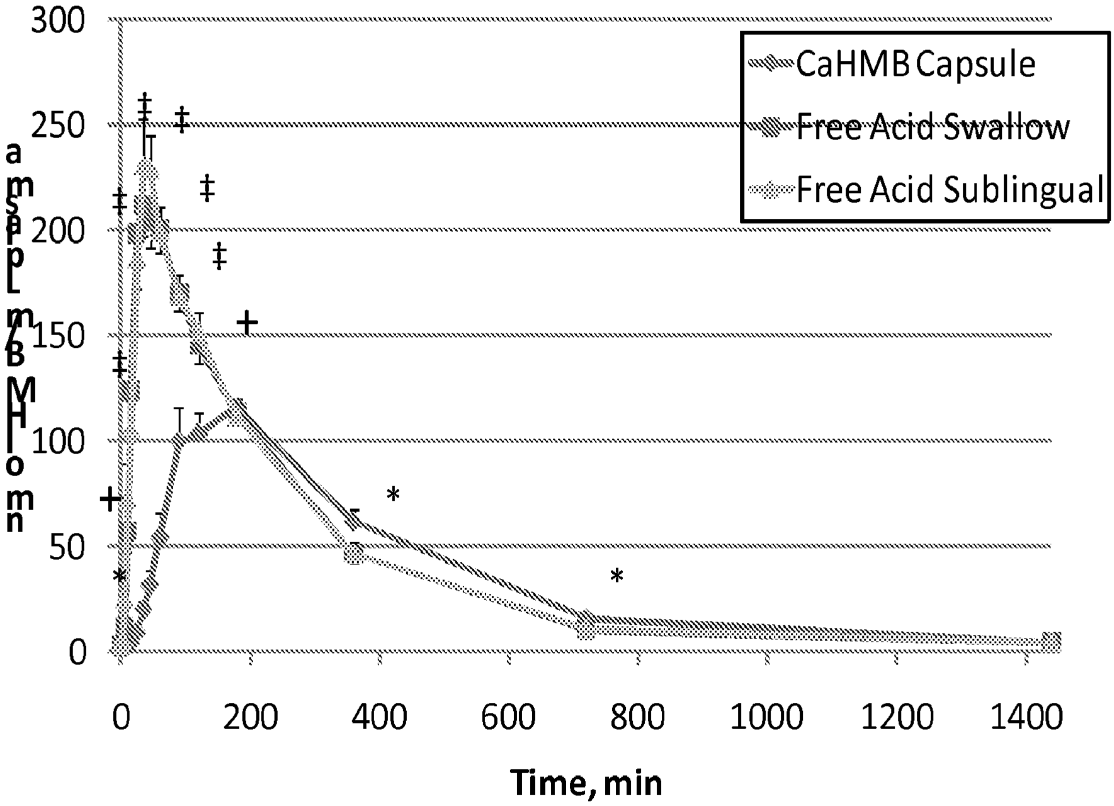
CaHMB Capsule
Free Acid Swallow
Free Acid Sublingual
0
50
100
150
200
250
300
0
200
400
600
800
1000
1200
1400
Time, min

FIG. 5

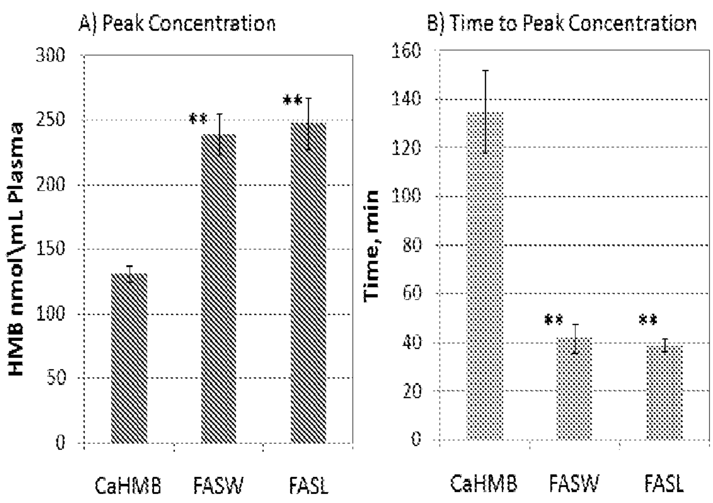
A) Peak Concentration
HMB nmol\mL Plasma
300
250
200
150
100
50
0
CaHMB
FASW
FASL
B) Time to Peak Concentration
Time, min
160
140
120
100
80
60
40
20
0
CaHMB
FASW
FASL

FIG. 6

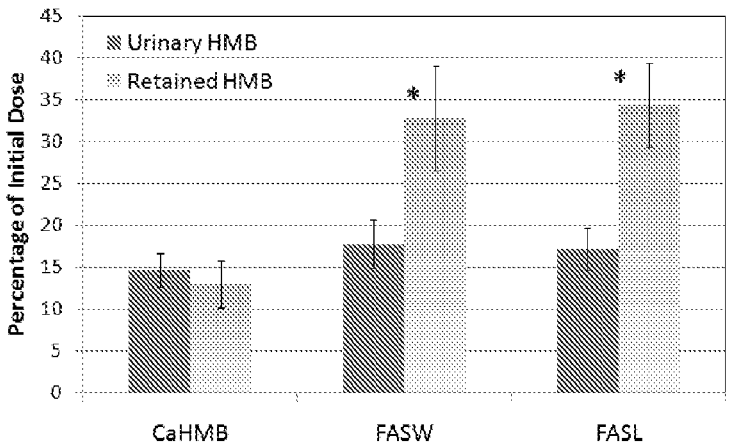
45
40
35
30
25
20
15
10
5
0
Percentage of Initial Dose
Urinary HMB
Retained HMB
*
*
CaHMB
FASW
FASL

FIG. 7

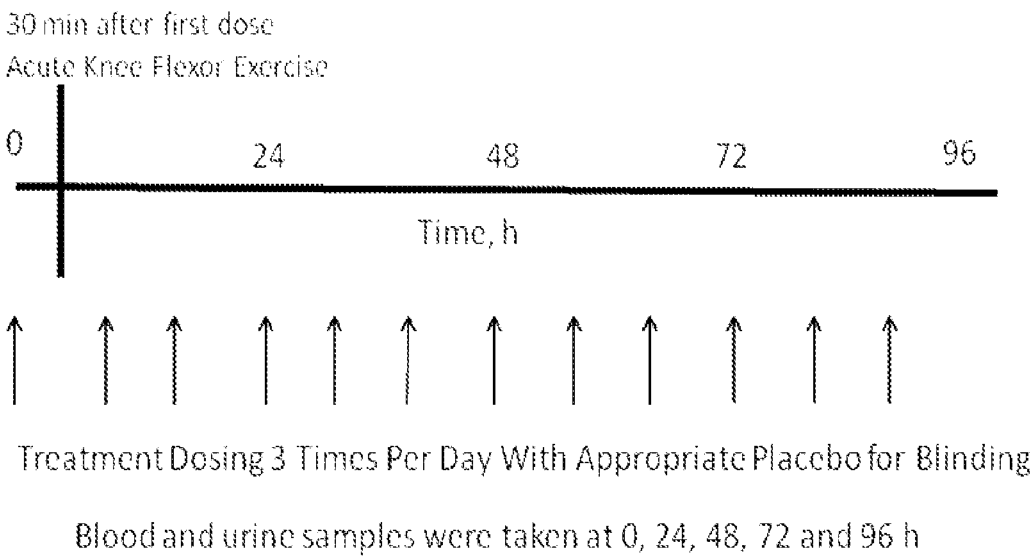
30 min after first dose
Acute Knee Flexor Exercise
0
24
48
72
96
Time, h
Treatment Dosing 3 Times Per Day With Appropriate Placebo for Blinding
Blood and urine samples were taken at 0, 24, 48, 72 and 96 h

FIG. 8

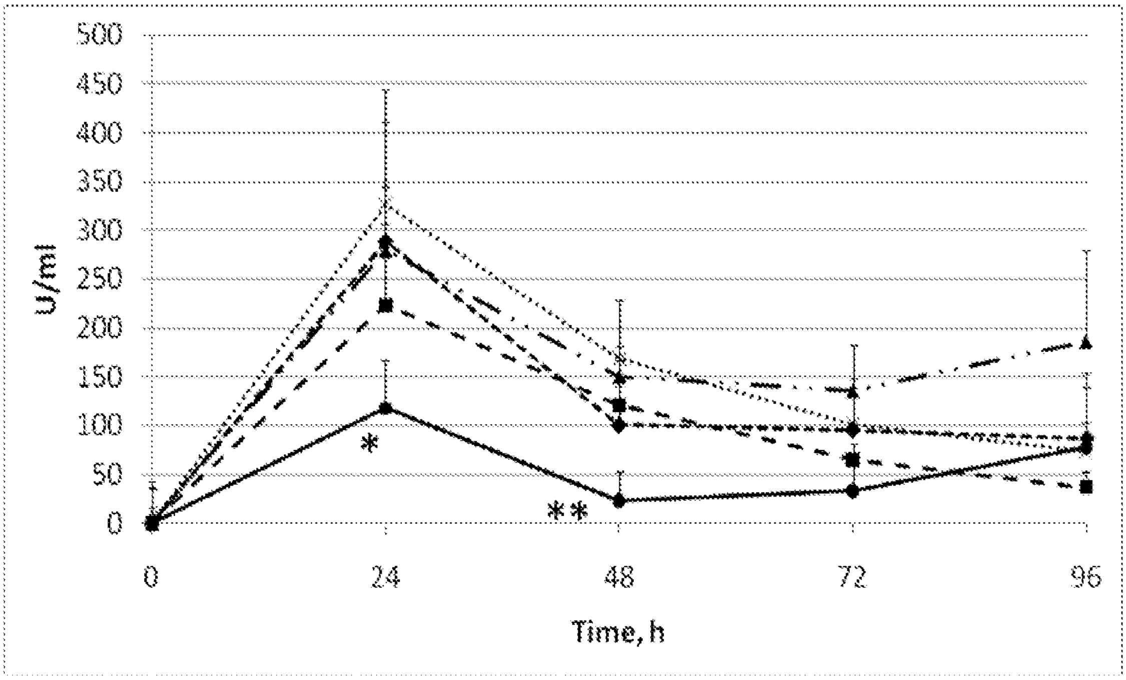


**FIG 9. Change in serum creatine phosphokinase (CPK) after an acute bout of eccentric exercise.** **-- ■ --** placebo, - ▲ - Calcium HMB pre-, -X- HMB free acid gel pre-, -♦- Calcium HMB pre- and post exercise, and **-●-** HMB free acid gel pre- and post exercise. * $P < 0.03$ contrasted to all other treatments; ** $P < 0.005$ contrasted to all other treatments and $P < 0.06$ contrasted with CaHMB alone and Placebo alone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61287857 B
- US 5360613 A
- US 5348979 A, Nissen
- US 5028440 A
- US 4992470 A
- US 6031000 A, Nissen
- US 6103764 A
- US 6291525 B


### Non-patent literature cited in the description


- **ADAMSON LF ; GREENBERG DM.** The significance of certain carboxylic acids as intermediates in the biosynthesis of cholesterol. *Biochim Biophys Acta,* 1957, vol. 23, 472-479
- **BACHHAWAT BK ; ROBINSON WG ; COON MJ.** The enzymatic cleavage of beta-hydroxy-beta-methylglutaryl coenzyme a to aceto-acetate and acetyl coenzyme A. *J Biol Chem,* 1955, vol. 216, 727-736
- **BLOCH K ; CLARK LC ; HARAY I.** Utilization of branched chain acids in cholesterol synthesis. *J Biol Chem,* 1954, vol. 211, 687-699
- **CLARK RH ; FELEKE G ; DIN M ; YASMIN T ; SINGH G ; KHAN F ; RATHMACHER JA.** Nutritional treatment for acquired immunodeficiency virus-associated wasting using β-hydroxy-β-methylbutyrate, glutamine and arginine: A randomized, double-blind, placebo-controlled study. *JPEN J Parenter Enteral Nutr,* 2000, vol. 24 (3), 133-139
- **COON MJ.** Enzymatic synthesis of branched chain acids from amino acids. *Fed Proc,* 1955, vol. 14, 762-764
- **DREYER HC ; DRUMMOND MJ ; PENNINGS B ; FUJITA S ; GLYNN EL ; CHINKES DL ; DHANANI S ; VOLPI E ; RASMUSSEN BB.** Leucine-enriched essential amino acid and carbohydrate ingestion following resistance exercise enhances mTOR signaling and protein synthesis in human muscle. *Am J Plysiol Endocrinol Metab,* 2008, vol. 294, E392-E400
- **DREYER HC ; FUJITA S ; CADENAS JG ; CHINKES DL ; VOLPI E ; RASMUSSEN BB.** Resistance exercise increases AMPK activity and reduces 4E-BP1 phosphorylation and protein synthesis in human skeletal muscle. *J Plysiol,* 2006, vol. 576, 613-624
- **ELEY HL ; RUSSELL ST ; BAXTER JH ; MUKHERJI P ; TISDALE MJ.** Signaling pathways initiated by β-hydroxy-β-methylbutyrate to attenuate the depression of protein synthesis in skeletal muscle in response to cachectic stimuli. *Am J Plysiol Endocrinol Metab,* 2007, vol. 293, E923-E931
- **ELEY HL ; RUSSELL ST ; TISDALE MJ.** Attenuation of depression of muscle protein synthesis induced by lipopolysaccharide, tumor necrosis factor and angiotensin II by β-hydroxy-β-methylbutyrate. *Am J Physiol Endocrinol Metab,* 2008, vol. 295, E1409-E1416
- **ELEY HL ; RUSSELL ST ; TISDALE MJ.** Mechanism of Attenuation of Muscle Protein Degradation Induced by Tumor Necrosis Factor Alpha and Angiotensin II by beta-Hydroxy-beta-methylbutyrate. *Am J Physiol Endocrinol Metab,* 2008, vol. 295, E1417-E1426
- **EUBANKS MAY P ; BARBER A ; HOURIHANE A ; D'OLIMPIO JT ; ABUMRAD NN.** Reversal of cancer-related wasting using oral supplementation with a combination of β-hydroxy-β-methylbutyrate, arginine, and glutamine. *Am J Surg,* 2002, vol. 183, 471-479
- Free acid gel form of β-hydroxy-β-methylbutyrate (HMB) improves HMB clearance from plasma in humans compared to the calcium HMB salt. **FULLER, J. C., JR. ; SHARP, R. L. ; ANGUS, H. F. ; BAIER, S. M. ; RATHMACHER, J. A.** Br.J.Nutr. 2010. Ref Type. In Press
- **GALLAGHER PM ; CARRITHERS JA ; GODARD MP ; SCHULZE KE ; TRAPPE SW.** β-Hydroxy-β-methylbutyrate ingestion, Part I: Effects on strength and fat free mass. *Med Sci Sports Exerc,* 2000, vol. 32 (12), 2109-2115
- **GALLAGHER PM ; CARRITHERS JA ; GODARD MP ; SCHUTZE KE ; TRAPPE SW.** β-Hydroxy-β-methylbutyrate ingestion, Part II: Effects on hematology, hepatic, and renal function. *Med Sci Sports Exerc,* 2000, vol. 32 (12), 2116-2119

- **GEY KF ; PLETSHER A ; ISLER O ; RUEGG R ; WURSCH J.** Influence of iosoprenoid C5 and C6 compounds on the incorporation of acetate in cholesterol. *Helvetica Chim Acta,* 1957, vol. 40, 2354-2368
- **GEY, K. F. ; PLETSHER, A. ; ISLER, O. ; RUEGG, R. ; WURSCH, J.** The influence of isoperenic C5 and C6 compounds upon the acetate incorporation into cholesterol. *Helvetica Chim.Acta,* 1957, vol. 40, 2369
- **HARPER AE ; BENEVENGA NJ ; WOHLHUETER RM.** Effects of ingestion of disproportionate amounts of amino acids. *Physiol Rev,* 1970, vol. 53, 428-558
- **HILL, D. S. ; SZEWCZYK, N. ; BROOKFIELD, R. ; LOUGHNA, P. ; RATHMACHER, J. ; RENNIE, M. J. ; ATHERTON, P. J.** β-hydroxy-β-methylbutyrate (HMB) stimulates mammalian target of rapamycin complex 1 (mTORc1) signaling via a phosphatidylinositol-3-kinase (PI3K)-dependent pathway. *FASEB J.,* 2011
- **ISLER, O. ; RUEGG, R. ; WURSCH, J. ; GEY, K. F. ; PLETSHER, A.** Biosynthesis of cholesterol from β,τ-dihydroxy-β-methylvaleric acid. *Helvetica Chim.Acta,* 1957, vol. 40, 2369
- **JANSSEN GME ; KUIPERS H ; WILLEMS GM ; DOES RJMM ; JANSSEN MPE ; GEURTEN P.** Plasma activity of muscle enzymes: quantification of skeletal muscle damage and relationship with metabolic variables. *Int J Sport,* 1989, vol. 10, S160-S168
- **JÓWKO E ; OSTASZEWSKI P ; JANK M ; SACHARUK J ; ZIENIEWICZ A ; WILCZAK J ; NISSEN S.** Creatine and β-hydroxy-β-methylbutyrate (HMB) additively increases lean body mass and muscle strength during a weight training program. *Nutr,* 2001, vol. 17, 558-566
- **KNITTER AE ; PANTON L ; RATHMACHER JA ; PETERSEN A ; SHARP R.** Effects of β-hydroxy-β-methylbutyrate on muscle damage following a prolonged run. *J Appl Physiol,* 2000, vol. 89 (4), 1340-1344
- **KREBS HA ; LUND P.** Aspects of the regulation of the metabolism of branched-chain amino acids. *Advan Enzyme Regul,* 1977, vol. 15, 375-394
- **NISSEN S ; PANTON L ; SHARP RL ; VUKOVICH M ; TRAPPE SW ; FULLER JC, JR.** β-Hydroxy-β-methylbutyrate (HMB) supplementation in humans is safe and may decrease cardiovascular risk factors. *J Nutr,* 2000, vol. 130, 1937-1945
- **NISSEN S ; SHARP R ; RAY M ; RATHMACHER JA ; RICE J ; FULLER JC, JR. ; CONNELLY AS ; ABUMRAD NN.** Effect of the leucine metabolite β-hydroxy β-methylbutyrate on muscle metabolism during resistance-exercise training. *JAppl Plysiol,* 1996, vol. 81 (5), 2095-2104
- **NISSEN S ; VAN KOEVERING M ; WEBB D.** Analysis of β-Hydroxy-β-methyl Butyrate in Plasma by Gas Chromatography and Mass Spectrometry. *Anal Biochem,* 1990, vol. 188 (1), 17-19
- **NISSEN S ; VAN KOEVERING M ; WEBB D.** Analysis of β-hydroxy-β-methyl butyrate in plasma by gas chromatography and mass spectrometry. *Anal Biochem,* 1990, vol. 188, 17-19
- **NISSEN SL ; ABUMRAD NN.** Nutritional role of the leucine metabolite β-hydroxy-β-methylbutyrate (HMB). *J Nutr Biochem,* 1997, vol. 8, 300-311
- **NISSEN SL ; SHARP RL.** Effect of dietary supplements on lean mass and strength gains with resistance exercise: a meta-analysis. *J Appl Plysiol,* 2003, vol. 94, 651-659
- **NUVIALA RJ ; RODA L ; LAPIEZA MG ; BONED B ; GINER A.** Serum enzymes activities at rest and after a marathon race. *J Sports Med Phys Fitness,* 1992, vol. 32, 180-186
- **OSTASZEWSKI P ; KOSTIUK S ; BALASINSKA B ; JANK M ; PAPET I ; GLOMOT F.** The leucine metabolite 3-hydroxy-3-methylbutyrate (HMB) modifies protein turnover in muscles of the laboratory rats and domestic chicken in vitro. *J Anim Plysiol Anim Nutr (Swiss),* 2000, vol. 84, 1-8
- **PANTON LB ; RATHMACHER JA ; BAIER S ; NISSEN S.** Nutritional supplementation of the leucine metabolite β-hydroxy β-methylbutyrate (HMB) during resistance training. *Nutr,* 2000, vol. 16 (9), 734-739
- **PHILLIPS SM ; TIPTON KD ; AARSLAND A ; WOLF SE ; WOLFE RR.** Mixed muscle protein synthesis and breakdown after resistance exercise in humans. *Am J Physiol,* 1997, vol. 273, E99-107
- **PLAUT GWE ; LARDY HA.** Enzymatic incorporation of C14-bicarbonate into acetoacetate in the presence of various substrates. *J Biol Chem,* 1951, vol. 192, 435-445
- **RABINOWITZ JL ; DITURI F ; COBEY F ; GURIN S.** Branched chain acids in the biosynthesis of squalene and cholesterol. *Fed Proc,* 1955, vol. 14, 760-761
- **RATHMACHER JA ; LINK GA ; FLAKOLL PJ ; NISSEN SL.** Gas chromatographic-mass spectrometric analysis of stable isotopes of 3-methylhistidine in biological fluids: application to plasma kinetics in vivo. *Biol Mass Spectrom,* 1992, vol. 21, 560-566
- **RATHMACHER JA ; NISSEN S ; PANTON L ; CLARK RH ; EUBANKS MP ; BARBER AE ; D'OLIMPIO J ; ABUMRAD NN.** Supplementation with a combination of beta-hydroxy-beta-methylbutyrate (HMB), arginine, and glutamine is safe and could improve hematological parameters. *JPEN J Parenter Enteral Nutr,* 2004, vol. 28, 65-75
- **RATHMACHER JA ; NISSEN SL.** Development and application of a compartmental model of 3-methylhistidine metabolism in humans and domestic animals. *Adv Exp Med Biol,* 1998, vol. 445, 303-324

- **RENNIE MJ.** Exercise- and nutrient-controlled mechanisms involved in maintenance of the musculoskeletal mass. *Biochem Soc Trans,* 2007, vol. 35, 1302-1305
- **ROBINSON WG ; BACHHAWAT BK ; COON MJ.** Enzymatic carbon dioxide fixation by senecioyl coenzyme A. *Fed Proc,* 1954, vol. 13, 281
- **RUDNEY H.** The synthesis of β-hydroxy-β-methylglutaric acid in rat liver homogenates. *J Am Chem Soc,* 1954, vol. 76, 2595
- **RUDNEY H ; FARKAS TG.** Biosynthesis of branched chain acids. *Fed Proc September,* 1955, 757-759
- **RUSSELL ST ; TISDALE MJ.** Mechanism of attenuation by beta-hydroxy-beta-methylbutyrate of muscle protein degradation induced by lipopolysaccharide. *Mol Cell Biochem,* vol. 330 (1-2), 171-179, 2009
- **SLATER G ; JENKINS D ; LOGAN P ; LEE H ; VUKOVICH MD ; RATHMACHER JA ; HAHN AG.** b-hydroxy b-methylbutyrate (HMB) supplementation does not affect changes in strength or body composition during resistance training in trained men. *Int J Sport Nutr Exerc Metab,* 2001, vol. 11, 384-396
- **SMITH HJ ; MUKERJI P ; TISDALE MJ.** Attenuation of proteasome-induced proteolysis in skeletal muscle by β-hydroxy-β-methylbutyrate in cancer-induced muscle loss. *Cancer Res,* 2005, vol. 65, 277-283
- **SMITH HJ ; WYKE SM ; TISDALE MJ.** Mechanism of the attenuation of proteolysis-inducing factor stimulated protein degradation in muscle by beta-hydroxy-beta-methylbutyrate. *Cancer Res,* 2004, vol. 64, 8731-8735
- **SOUSA MF ; ABUMRAD NN ; MARTINS C ; NISSEN S ; RIELLA MC.** Calcium β-hydroxy-β-methylbutyrate. Potential role as a phosphate binder in uremia: In vitro study. *Nephron,* 1996, vol. 72, 391-394
- **THALHAMMER F ; SCHENK P ; BURGMANN H ; EI M, I ; HOLLENSTEIN UM ; ROSENKRANZ AR ; SUNDER-PLASSMANN G ; BREYER S ; RATHEISER K.** Single-dose pharmacokinetics of meropenem during continuous venovenous hemofiltration. *Antimicrob Agents Chemother,* 1998, vol. 42, 2417-2420
- **URSO R ; BLARDI P ; GIORGI G.** A short introduction to pharmacokinetics. *Eur Rev Med Pharmacol Sci,* 2002, vol. 6, 33-44
- **VUKOVICH MD ; SLATER G ; MACCHI MB ; TURNER MJ ; FALLON K ; BOSTON T ; RATHMACHER J.** β-Hydroxy-β-methylbutyrate (HMB) kinetics and the influence of glucose ingestion in humans. *J Nutr Biochem,* 2001, vol. 12, 631-639
- **VUKOVICH MD ; STUBBS NB ; BOHLKEN RM.** Body composition in 70-year old adults responds to dietary β-hydroxy-β-methylbutyrate (HMB) similar to that of young adults. *J Nutr,* 2001, vol. 131 (7), 2049-2052
- **ZABIN I ; BLOCH K.** The utilization of butyric acid for the synthesis of cholesterol and fatty acids. *J Biol Chem,* 1951, vol. 192, 261-266